(19) Europäisches Patentamt
European Patent Office
Office européen des brevets

(11) **EP 4 453 864 B1**

(12) **EUROPEAN PATENT SPECIFICATION**

(45) Date of publication and mention
of the grant of the patent:
**12.11.2025   Bulletin 2025/46**

(21) Application number: **22868459.3**

(22) Date of filing: **22.12.2022**

(51) International Patent Classification (IPC):
*G06T 7/00* (2017.01)        *G06V 10/25* (2022.01)

(52) Cooperative Patent Classification (CPC):
**G06T 7/0012; G06V 10/25; G06V 10/255;**
**G06V 10/82;** G06T 2207/10132; G06T 2207/20081;
G06T 2207/20084; G06T 2207/30084;
G06T 2207/30096; G06V 2201/031

(86) International application number:
**PCT/IB2022/000779**

(87) International publication number:
**WO 2023/118964 (29.06.2023 Gazette 2023/26)**

(54) **AUTOMATED ULTRASOUND IMAGING ANALYSIS AND FEEDBACK**

AUTOMATISIERTE ULTRASCHALLBILDGEBUNGSANALYSE UND RÜCKKOPPLUNG

ANALYSE ET RÉTROACTION D'IMAGERIE ULTRASONORE AUTOMATISÉE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.12.2021   US 202163292764 P**
**16.02.2022   US 202263310761 P**

(43) Date of publication of application:
**30.10.2024   Bulletin 2024/44**

(73) Proprietor: **Geonomy, Ltd.**
**68100 Mulhouse (FR)**

(72) Inventors:
• **KOLMER, Sebastien**
**68100 Mulhouse (FR)**
• **SCHERER, Stefan**
**Jersey City, NJ 07302 (US)**
• **MIQUEL, Francois**
**68100 Mulhouse (FR)**

(74) Representative: **HGF**
**HGF Limited**
**1 City Walk**
**Leeds LS11 9DX (GB)**

(56) References cited:
**CN-A- 110 599 476     CN-A- 111 754 530**
**CN-A- 112 270 682**

**Description**

**Cross-Reference to Related Applications**

This application claims priority to, and the benefit of, U.S. Provisional Application No. 63/292,764, filed December 22, 2021, and U.S. Provisional Application No. 63/310,761, filed February 16, 2022.

**Technical Field**

The invention generally relates to medical imaging, and, more particularly, to a digital platform providing automated ultrasound image interpretation and analysis, including providing augmented image feedback based on artificial intelligence techniques.

**Background**

Medical imaging refers to several different technologies that are used to view the human body in order to diagnose, monitor, or treat medical conditions. As such, medical imaging is generally recognized as one of the most powerful diagnostic and intervention tools in medicine. The most common types of medical imaging modalities include, but are not limited to, x-ray imaging, Magnetic Resonance Imaging (MRI), and ultrasound (US) imaging. While each type of imaging modality has its particular advantages, as well as its associated drawbacks, ultrasound is becoming a more common imaging technique, due in large part to its portability, ease of use, noninvasiveness, and reduced costs, when compared to other imaging modalities.

Ultrasound imaging is a medical imaging technique for imaging organs and soft tissues in a human body. Ultrasound imaging uses real time, non-invasive high frequency sound waves to produce a two-dimensional (2D) image and/or a three-dimensional (3D) image. The ultrasound image is produced based on the reflection of the waves off of the body structures. The strength (amplitude) of the sound signal and the time it takes for the wave to travel through the body provide the information necessary to produce an image. Ultrasound imaging can help a physician evaluate, diagnose and treat various medical conditions.

When making a diagnosis based on an ultrasound examination, physicians must rely on adequate image quality, acquisition of proper views, and sufficient quantification of all relevant structures and flows. Although image quality is usually constant within a system and acquisition of proper views is typically associated with a standard protocol within each lab, quantification of all relevant information is particularly problematic.

For example, in current ultrasound systems, the operator must have sufficient knowledge of the structures, as well as any associated flows (i.e., flow of fluid, such as blood, within associated structures), associated with various diagnoses to interpret the results of the ultrasound measurements. In particular, unlike other imaging modalities which rely on a more objective and standard examination and analysis (such as an MRI scan), an ultrasound examination is highly operator dependent, and the acquisition of the image and its interpretation is entirely dependent on operator's dexterity in handling an ultrasound probe and the operator's capacity to distinguish the elements present in the image. With regard to certain procedures, such as a point of care ultrasonography, unlike a radiologist, whose core business consists of performing a full examination for interpreting an image, the operator or clinician of the ultrasound examination is seeking an immediate response to a definite and generally binary diagnostic problem that they are familiar with. Moreover, such point of care diagnostics may require iteration of ultrasound examinations concerned with this specific problem.

Furthermore, ultrasound imaging poses certain technical challenges, including, but not limited to, a significant loss of information during the reconstruction of the signal, a dependency of the signal from the direction of acquisition, and a speckle noise that corrupts the resulting image and affects the interpretation of the anatomical area during the course of the examination, which further necessitates analysis of video or a sequence of images, rather than fixed images. Also, the acquisition and analysis of ultrasound images must generally be performed simultaneously, such that the patient and operator must both be present during the examination. Accordingly, the value of diagnosis is entirely dependent on the operator, specifically their knowledge, skill, and availability.

It is known from CN 110 599 476 to provide a disease grading method and device based on machine learning. CN 111 754 530 provides a prostate ultrasonic segmentation and classification method.

**Summary**

The present invention recognizes the drawbacks of ultrasound imaging, particularly the constraints associated with ultrasound image analysis and diagnosis, and provides an automated ultrasound image interpretation and analysis platform to address such drawbacks. The invention is defined by the appended claims.

Aspects of the invention may be accomplished using a platform configured to analyze ultrasound images acquired during an examination and, in turn, provide augmented image feedback based on artificial intelligence techniques. The image feedback is generally in the form of an augmented ultrasound image provided to a display associated with the ultrasound imaging machine.

In particular, the invention provides a computing system as disclosed in appended claim 1.

The computing system is configured to receive ultrasound image data, which includes ultrasound images of a target site of a patient having undergone an ultrasound examination. Upon receiving the sample ultrasound images, the computing system is configured to analyze the ultrasound images using the neural network and based on an association of the condition data with the reference ultrasound image data. Based on such analysis, the computing system is able to identify types of tissue and/or anatomical structures within the ultrasound image and further identify a condition associated with any types of tissue and/or anatomical structures identified. In turn, an augmented ultrasound image can be provided, via a display associated with an ultrasound machine or associated with a computing device.

The augmented ultrasound image may generally include a visual representation of one or more identified types of tissue and/or anatomical structures at a target site of a patient that has undergone ultrasound image examination, as well any identified abnormal conditions. In particular, the visual representation includes a first layer of content overlaid upon ultrasound images which provides semantic segmentation of different types of tissue and/or anatomical structures (i.e., organs or the like). For example, the first layer may include shaded or patterned zones that are overlaid upon portions of the ultrasound image, as well as associated text indicating each shaded zone represents identified tissue types and/or anatomical structures within the image. The shaded or patterned zones may each have a different color relative to one another to enhance visual distinction of the different tissue and/or structures from one another. The ultrasound image may further be augmented with a second layer of content that provides an indication of any abnormal condition or anomaly associated with the identified tissues and/or anatomical structures. For example, in the event that the automated analysis identifies an abnormal condition, a second layer is overlaid upon the ultrasound image, which includes a visual indication of the abnormal condition associated with the one or more identified types of tissue and/or identified anatomical structures. The second layer may include, for example, a marking and/or shaded zone corresponding to at least a portion of one of the identified types of tissue and/or identified anatomical structures to which the abnormal condition is associated, as well as text identifying the specific abnormal condition. The abnormal condition may include, for example, a disease or anomaly requiring medical treatment or the like.

It should be noted that the platform of the present invention may either be incorporated directly with an ultrasound machine (i.e., provided as a local component to an ultrasound imaging machine) or may be cloud-based and provide a digital, web-based application that an operator can access via an ultrasound machine or computing device (i.e., smartphone, tablet, personal computer, or the like).

As such, in some embodiments, in which the platform is embedded within an imaging machine, the platform is configured to provide real, or near-real, time augmented feedback to an operator of the imaging machine. In particular, the platform is configured to analyze the ultrasound images acquired by the imaging machine during an examination. Based on that analysis, the platform is able to then provide the augmented image feedback to the operator while the examination is occurring, which is generally in the form of an augmented ultrasound image that is presented to the operator in real, or near-real, time during the examination.

In embodiments in which the platform is cloud-based, such as a software as a service (SaaS) model or the like, the platform is able receive images acquired by any contemplated imaging machine, such as any typical ultrasound imaging machine, and analyze those standard ultrasound images in a deferred manner. In other words, an ultrasound examination may already have been completed, and the ultrasound images from the examination have been saved for later interpretation. Accordingly, an operator need only upload the saved images of interest to the cloud-based platform (i.e., via a computing device, such as a computing device associated with the imaging machine, a smartphone, a tablet, a laptop, or the like). In turn, the platform is configured to analyze the ultrasound images, as previously described herein, and based on that analysis, the platform is able to provide augmented image feedback to the operator, in which the operator may view the augmented ultrasound images on their computing device.

Accordingly, by providing automated ultrasound image interpretation and analysis, and further providing augmented image feedback based on artificial intelligence techniques, the present invention addresses the limitations of current ultrasound imaging examinations, namely the constraints associated with interpreting ultrasound images and making a diagnosis. More specifically, unlike a traditional ultrasound examination, which requires the operator to have sufficient knowledge and practice in order to make an accurate diagnosis, the present invention reduces the need for such qualification and training. Rather, any clinician associated with a patient's care and with a minimum amount of training can perform ultrasound imaging and utilize the present invention to receive highly accurate analysis and interpretation of a target site having undergone examination. Additionally, the present invention further improves the ability of trained operators and clinicians (i.e., radiologists and the like) to interpret and diagnosis ultrasound images, in which the augmented feedback allows such users to better establish prognosis and a decision path for certain complex diagnostics, and further uncovering underlying biological features that may not be readily apparent.

Furthermore, by providing the augmented feedback, the present invention provides fundamental information to an operator, including a clear indication of the area of the patient that was imaged, a clear indication and segmentation of various structures captured in the images, and a clear indication of whether such structures are normal or abnormal and require medical intervention. As such, the present invention is able to process ultrasound images based off any standard image, regardless of origin and without modifying the standard ultrasound imaging procedure, thereby improving overall access to ultrasound examinations with a lower level of training and practice, while maintaining high diagnostic efficiency. In particular, the present invention is compatible with most standard and current imaging machines, in that the platform of the present invention analyzes ultrasound images that have already been acquired and reconstructed by the imaging machine and is not required for such image reconstruction.

In one aspect, a system for providing automated medical imaging analysis is provided. The system includes a computing system comprising a hardware processor coupled to non-transitory, computer-readable memory containing instructions executable by the processor to cause the computing system to perform various operations for analyzing ultrasound images and providing augmented feedback to an operator.

In particular, the system is configured to run a neural network, wherein the neural network has been trained using a plurality of training data sets, each training data set comprises reference ultrasound image data associated with known tissue and/or anatomical structures and known condition data associated with the known tissue and/or anatomical structures. The system is configured to receive and analyze a sample ultrasound image of a target site of a patient by using the neural network and based on an association of the condition data with the reference ultrasound image data. The analysis of the sample ultrasound image comprises correlating sample image data with the known tissue and/or anatomical structures of the reference ultrasound image data and known condition data associated therewith.

The system is further configured to identify, based on the analysis, one or more types of tissue and/or anatomical structures and an associated condition of the one or more types of tissue and/or anatomical structures in the sample ultrasound image. The identified types of tissue and/or anatomical structures may include, but are not limited to, muscles, bones, organs, blood vessels, and nerves. The condition may include, for example, either a normal condition, which is indicative of an otherwise health state, or an abnormal condition, which may be indicative of damage and or a disease requiring medical intervention.

In turn, the system is configured to output, via a display, an augmented ultrasound image comprising a visual representation of the one or more identified types of tissue and/or anatomical structures at the target site of the patient and an associated abnormal condition of the one or more identified types of tissue and/or anatomical structures, if present.

The computing system includes a machine learning system selected from the group consisting of a neural network, a random forest, a support vector machine, a Bayesian classifier, a Hidden Markov model, an independent component analysis method, and a clustering method.

In some embodiments, the computing system comprises an autonomous machine learning system that associates the condition data with the reference ultrasound image data. For example, the machine learning system may include a deep learning neural network that includes an input layer, a plurality of hidden layers, and an output layer. The autonomous machine learning system may represent the training data set using a plurality of features, wherein each feature comprises a feature vector. For example, the autonomous machine learning system may include a convolutional neural network (CNN).

It should be noted that, in some embodiments, the reference ultrasound image data and condition data are obtained from one or more third party sources. The third party sources may include publicly available or subscription-based data sources.

In some embodiments, the visual representation comprises at least a first layer of content overlaid upon the sample ultrasound image. For example, the first layer of content comprises semantic segmentation of different types of tissue within the sample ultrasound image and/or different anatomical structures within the sample ultrasound image. The first layer of content may include one or more shaded zones overlaid upon one or more respective portions of the sample ultrasound image. Each of the one or more shaded zones corresponds to a respective one of the identified types of tissue and/or identified anatomical structures. Each of the identified types of tissue and/or identified anatomical structures comprises a shaded zone with a distinct color and/or pattern to distinguish from one another. The first layer of content may further include text associated with each of the one or more shaded zones, wherein the text identifies the respective one of the identified types of tissue and/or identified anatomical structures.

The visual representation may also include a second layer of content overlaid upon the sample ultrasound image. The second layer of content comprises a visual indication of an abnormal condition associated with the one or more identified types of tissue and/or identified anatomical structures. The visual indication may include text identifying the specific abnormal condition. The visual indication may also include a marking and/or shaded zone corresponding to at least a portion of one of the identified types of tissue and/or identified anatomical structures to which the abnormal condition is associated.

**Brief Description of the Drawings**

FIGS. 1A and 1B are diagrammatic illustrations of a conventional ultrasound imaging system compatible for use with a system for providing automated ultrasound image interpretation and analysis consistent with the present disclosure.

FIG. 2 is a block diagram illustrating a system for providing automated ultrasound image interpretation and analysis consistent with the present disclosure.

FIG. 3 is a block diagram illustrating an automated ultrasound imaging analysis system, including a machine learning system, consistent with the present disclosure.

FIG. 4 is a block diagram illustrating inputting of reference data (i.e., training data sets) into the machine learning system.

FIG. 5 shows a machine learning system according to certain embodiments of the present disclosure.

FIG. 6 is a block diagram illustrating one embodiment of a model illustrating transfer learning training of data sets for use in the automated ultrasound image interpretation and analysis consistent with the present disclosure.

FIG. 7 is a flow diagram illustrating one embodiment of a data qualification and verification process for use with reference ultrasound image data and condition data prior to incorporation into a trained data set of the machine learning system of the present invention.

FIG. 8 is an exemplary graph illustrating model performance based on the development of training and testing of datasets for use with the machine learning system of the present invention.

FIG. 9 is a block diagram illustrating receipt of one or more sample ultrasound images acquired during an ultrasound examination of a target site of a patient, subsequent processing of said sample ultrasound images via a machine learning system and image analysis and content creation module of the present disclosure, and outputting of ultrasound image overlay content to be displayed in an augmented ultrasound image.

FIG. 10A is an exemplary ultrasound image acquired during an ultrasound examination of a patient's lower abdominal area.

FIG. 10B is an exemplary augmented ultrasound image generated based, at least in part, on processing of the ultrasound image of FIG. 10A via the automated ultrasound imaging analysis system, including the machine learning system, consistent with the present disclosure. The augmented ultrasound image includes a first layer providing semantic segmentation of the various organs, including segmentation of the liver, gallbladder and digestive structure, as well as a second layer providing an indication of the presence of gallstones in the gallbladder.

FIG. 11 is an exemplary augmented ultrasound image generated based on the systems and methods of the present invention, which illustrates capabilities of providing automatic nerve localization associated with an ultrasound examination.

FIG. 12 is an exemplary augmented ultrasound image generated based on the systems and methods of the present invention, which illustrates capabilities of providing detection of abnormal growths of tissue (e.g., tumors of the like) within an ultrasound image and an indication of the likelihood that such growth is benign or malignant.

FIG. 13 is a block diagram illustrating one embodiment of a system consistent with the present invention, which is provided via an on-demand prediction service model.

FIG. 14 is a block diagram illustrating another embodiment of a system consistent with the present invention, which is provided locally and incorporated into an ultrasound machine.

FIG. 15 is a block diagram illustrating one embodiment of a process for collecting and subsequently labeling medical data for developing a machine learning model consistent with the present invention for providing anomaly detection.

FIGS. 16, 17, and 18 show rule sets for processing collected data (from ultrasound films) in accordance with the present disclosure.

FIGS. 19A and 19B are bar graphs illustrating prediction results and analysis of anomaly classification and normal classification, respectively, consistent with the systems and methods of the present disclosure.

FIG. 20 is a flow diagram illustrating the overall workflow of an anomaly detection framework consistent with the present disclosure.

FIGS. 21A and 21B are screenshots of an exemplary user interface of a web app illustrating a sample US film provided by the user and subsequent determination of various characteristics of the film based on processing/analysis performed via the anomaly detection machine learning model consistent with the present disclosure.

FIG. 22 is a flow diagram illustrating the process of collecting the required data for training a statistical model to automatically describe tissue and organs encountered during an ultrasound examination, specifically for anatomical topography via a semantic segmentation, including the automatic detection of axillary nerves for upper limb operations without general anesthesia, referred to as "nerve cartography".

FIG. 23 is an exemplary augmented ultrasound image generated based on the systems and methods of the present invention, which illustrates the conversion of polygon annotation objects into fully colored segmentation masks indicative of different organic structures.

FIG. 24 shows an exemplary architecture of a U-net++ type auto-encoder.

FIGS. 25A, 25B, and 25C are exemplary images of an original ultrasound film (FIG. 25A), a prediction given by the annotation model (i.e., a colorized mask for each type of class) (FIG. 25B), and a ground truth with the segmented classes following the annotation made by the professionals (FIG. 25C).

FIG. 26 is a bar graph illustrating the performance of the annotation model on a training dataset.

FIGS. 27A, 27B, 27C are ultrasound images in which the artery, median nerve, ulnar nerve, radial nerve and musculocutaneous nerve are identified and labeled via respective fully colored segmentation masks and the location of which are tracked (spatially and temporally) over different frames (i.e., FIG. 27A to FIG. 27B to FIG. 27C).

FIG. 28 is a bar graph illustrating the performance characteristics of different computational hardware.

FIG. 29 is a block diagram illustrating an automated labeling framework consistent with the present disclosure.

## Detailed Description

By way of overview, the present invention is directed to systems and methods for providing automated ultrasound image interpretation and analysis. More specifically, aspects of the invention may be accomplished using a platform configured to analyze ultrasound images acquired during an examination and, in turn, provide augmented image feedback based on artificial intelligence techniques.

In particular, the platform is configured to receive input in the form of sample ultrasound images from an ultrasound machine associated with a target site of a patient having undergone an ultrasound examination. The platform is configured to analyze the ultrasound images using a neural network, relying on pre-trained data sets containing reference ultrasound image data and condition data. Accordingly, based on a correlation of the sample ultrasound images with the reference data, the platform is configured to automatically identify specific types of tissue and/or anatomical structures in the sample ultrasound images and further identify whether any of the identified tissue and/or anatomical structures is presenting an abnormal condition (i.e., a tissue and/or anatomical structure exhibiting certain physical characteristics associated with damage or a disease state or other undesired condition requiring medical treatment).

The image feedback is generally in the form of an augmented ultrasound image displayed to an operator or other clinician utilizing the platform for evaluation and interpretation of the acquired ultrasound images. The augmented ultrasound image includes a visual representation of one or more identified types of tissue and/or anatomical structures at a target site of the patient that is either currently undergoing, or has already undergone, ultrasound image examination, as well any identified abnormal conditions. In particular, at least two different types of information can be superimposed over the sample ultrasound image and presented to the operator or any clinician utilizing the platform for image analysis and interpretation. More specifically, a first layer of information superimposed over the image includes a semantic segmentation of the different identified tissues or organs, which is visually presented to the operator (i.e., by way of text and/or specific shaded or otherwise highlighted zones overlaid upon respective portions of the image in which those tissues or organs are present). A second layer of information that may be superimposed over the image includes any detected anomaly or abnormal condition of a given tissue or organ if present (i.e., by way of text and/or specific shaded or highlighted zone overlaid upon that specific tissue or organ).

Accordingly, the present invention addresses the limitations of current ultrasound imaging examinations, namely the constraints associated with interpreting ultrasound images and making a diagnosis. More specifically, unlike a traditional ultrasound examination, which requires the operator to have sufficient knowledge and practice in order to make an accurate diagnosis, the present invention reduces the need for such qualification and training. Rather, any clinician associated with a patient's care and with a minimum amount of training can perform ultrasound imaging and utilize the present invention to receive highly accurate analysis and interpretation of a target site having undergone examination.

Furthermore, by providing the augmented feedback, the present invention provides fundamental information to an operator, including a clear indication of the area of the patient that was imaged, a clear indication and segmentation of various structures captured in the images, and a clear indication of whether such structures are normal or abnormal and require medical intervention. As such, the present invention is able to process ultrasound images based off any standard image, regardless of origin and without modifying the standard ultrasound imaging procedure, thereby improving overall access to ultrasound examinations with a lower level of training and practice, while maintaining high diagnostic efficiency. In particular, the present invention is compatible with most standard and current imaging machines, in that the platform of the present invention analyzes ultrasound images that have already been acquired and reconstructed by the imaging machine and is not required for such image reconstruction.

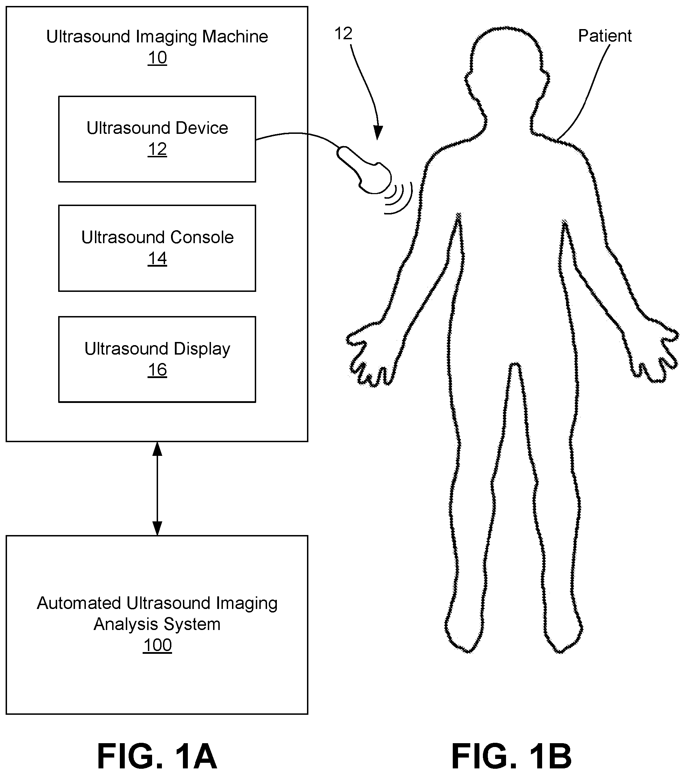

FIGS. 1A and 1B are diagrammatic illustrations of a conventional ultrasound imaging system 10 compatible for use with an automated ultrasound imaging analysis system 100 consistent with the present invention. The system 10 (also referred to herein as an ultrasound imaging machine 10) includes an ultrasound device 12, a console 14, and a display 16. As generally understood, ultrasound imaging (sonography) uses high-frequency sound waves to view inside the body. Because ultrasound images are captured in real-time, they can also show movement of the body's internal organs as well as fluid flow (e.g., blood flowing through blood vessels). In an ultrasound exam, the ultrasound device 12, also referred to as a transducer probe, is placed directly on the skin or inside a body opening. A thin layer of gel is applied to the skin so that the ultrasound waves are transmitted from the transducer through the gel into the body. The ultrasound transducer probe 12 is responsible for sending and receiving the sound waves that create an ultrasound image via the piezoelectric effect, a phenomenon that causes quartz crystals within the probe to vibrate rapidly and send out sound waves. These waves then bounce off objects and are reflected to the probe.

The transducer probe 12 is operably coupled to a console 14, which is generally controls operation of the transducer probe 12 (i.e., transmission of sound waves from the probe). The console 14 may generally include a central processing unit (CPU), storage, and some form of input (i.e., a keyboard, knobs, scroll wheels, or the like) with which an operator can interact so as to operate the machine, including making adjustments to the transmission characteristics of the probe, saving images, and performing other tasks. During operation, the CPU transmits electrical currents that cause the probe 12 to emit sound waves. The CPU also analyzes electrical pulses that the probe makes in response to reflected waves coming back. It then converts this data into images (i.e., ultrasound images) that can then be viewed on a display 16, which may be an integrated monitor. Such images may also be stored in memory and/or printed via a printer (not shown).

The present invention provides an automated ultrasound image interpretation and analysis platform. Aspects of the invention may be accomplished using a platform configured to analyze ultrasound images acquired during an examination and, in turn, provide augmented image feedback based on artificial intelligence techniques. The image feedback is generally in the form of an augmented ultrasound image provided to a display associated with the ultrasound imaging machine and/or a display of a computing device associated with an operator or clinician utilizing the platform of the present invention for image analysis and interpretation.

As shown in FIG. 1A, the invention may include an automated ultrasound imaging analysis system 100. This system 100 may either be incorporated directly with an ultrasound machine (i.e., provided as a local component to an ultrasound imaging machine) or may be cloud-based and provide a digital, web-based application that an operator can access via an ultrasound machine or computing device (i.e., smartphone, tablet, personal computer, or the like).

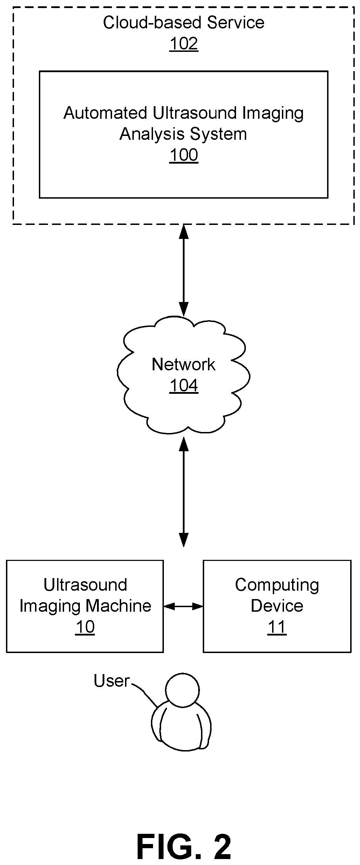

FIG. 2 is a block diagram illustrating the automated ultrasound imaging analysis system 100 in greater detail. As shown, the system 100 is embodied on a cloud-based service 102, for example. The automated ultrasound imaging analysis system 100 is configured to communicate and share data with an ultrasound imaging machine 10. It should be noted, however, that the system 100 may also be configured to communicate and share data with a computing device associated with a user. The computing device 11 may include a separate computer coupled to an ultrasound imaging machine. Yet still, in some embodiments, the computing device 11 may include a portable computing device, such as a smartphone, tablet, laptop computer, or the like. For example, advances in technology have led to some ultrasound probes being connectable to a personal and/or portable computing device. Accordingly, in some embodiments, the system 100 may be configured to communicate with an operator of an ultrasound probe via an associated smartphone or tablet. In the present context, the user may include a clinician, such as a physician, physician's assistant, nurse, or other medical professional that has provided an ultrasound examination on a patient and/or is otherwise associated with the medical care of the patient and is utilizing the platform of the present invention for ultrasound image evaluation and interpretation. The system 100 is configured to communicate and exchange data with the ultrasound imaging machine 10 and/or computing device 11 over a network 104, for example.

The network 104 may represent, for example, a private or non-private local area network (LAN), personal area network (PAN), storage area network (SAN), backbone network, global area network (GAN), wide area network (WAN), or collection of any such computer networks such as an intranet, extranet or the Internet (i.e., a global system of interconnected network upon which various applications or service run including, for example, the World Wide Web). In alternative embodiments, the communication path between the ultrasound imaging machine 10 and computing device 11 and/or between the machine 10, computing device 11 and system 100 may be, in whole or in part, a wired connection.

The network 104 may be any network that carries data. Non-limiting examples of suitable networks that may be used as network 18 include Wi-Fi wireless data communication technology, the internet, private networks, virtual private networks (VPN), public switch telephone networks (PSTN), integrated services digital networks (ISDN), digital subscriber link networks (DSL), various second generation (2G), third generation (3G), fourth generation (4G), fifth generation (5G), and future generations of cellular-based data communication technologies, Bluetooth radio, Near Field Communication (NFC), the most recently published versions of IEEE 802.11 transmission protocol standards, other networks capable of carrying data, and combinations thereof. In some embodiments, network 104 is chosen from the internet, at least one wireless network, at least one cellular telephone network, and combinations thereof. As such, the network 104 may include any number of additional devices, such as additional computers, routers, and switches, to facilitate communications. In some embodiments, the network 104 may be or include a single network, and in other embodiments the network 104 may be or include a collection of networks.

It should be noted that, in some embodiments, the system 100 is embedded directly into an ultrasound machine, or may be directly connected thereto in a local configuration, as opposed to providing a web-based application. For example, in some embodiments, the system 100 operates in communication with a medical setting, such as an examination or procedure room, laboratory, or the like, may be configured to communicate directly with instruments, including, for example, the ultrasound imaging machine 10 either via a wired or wireless connection.

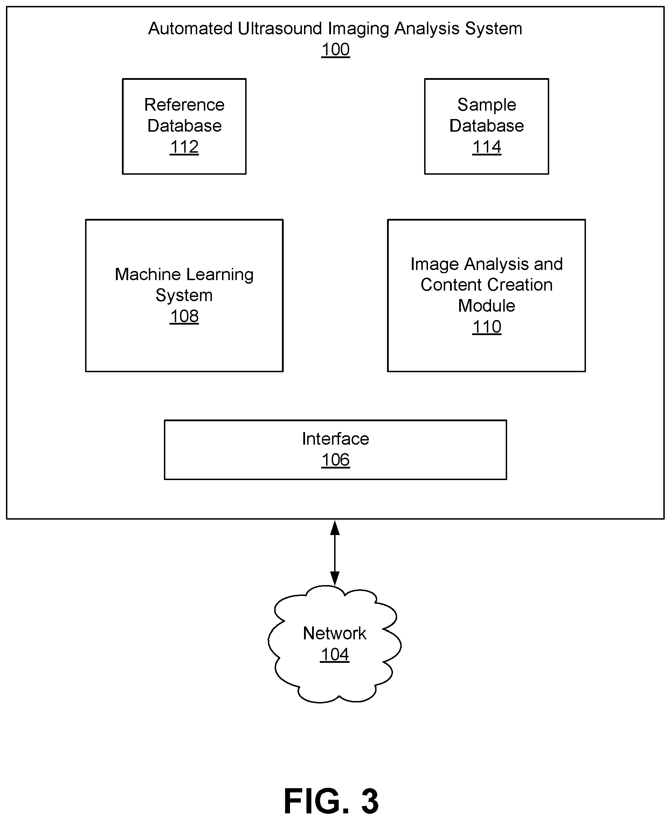

FIG. 3 is a block diagram illustrating an automated ultrasound imaging analysis system 100, including a machine learning system 108, for providing automated ultrasound analysis and feedback. The system 100 is preferably implemented in a tangible computer system built for implementing the various methods described herein.

As illustrated, the system 100 may generally be accessed by a user, to initiate methods of the invention and obtain results, via an interface 106, for example. The interface 106 allows for a user to connect with the platform provided via the system and provide sample ultrasound images to undergo automated analysis and feedback. The system 100 may further include one or more databases with which the machine learning system 108 communicates. In the present example, a reference database 112 includes stored reference data obtained from a plurality of training data sets and a sample database 114 includes stored sample data acquired as a result of evaluations carried out via the system 100 on sample ultrasound images. The system 100 further includes an image analysis and content creation module 110 for generating augmented feedback content based on analysis carried out by the machine learning system 108, as will be described in greater detail herein.

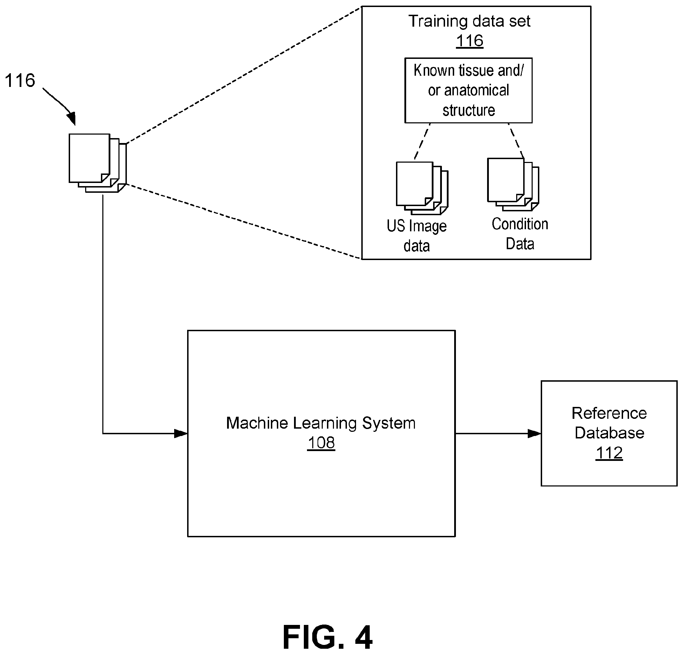

The system 100 generally runs a neural network that has been trained using a plurality of training data sets that include qualified reference data. FIG. 4 is a block diagram illustrating inputting of reference data (i.e., training data sets) into the machine learning system 108, for example. The machine learning techniques of the present invention, and the subsequent analysis of sample ultrasound images based on such techniques, utilize reference data. The reference data may include a plurality of training data sets 116 inputted to a machine learning system 108 of the present invention. For example, each training data set includes reference ultrasound image data, which may include, for example, ultrasound images that include known types of tissue and/or anatomical structures. Each training data set further includes known condition data associated with the known types of tissue and/or anatomical structures. The condition data may include, for example, a condition status of a known type of tissue or anatomical structure of a given reference ultrasound image. The condition status may include a normal condition (i.e., unremarkable or otherwise healthy condition for tissue and/or anatomical structure) or an abnormal condition (i.e., a tissue and/or anatomical structure exhibiting certain physical characteristics associated with damage or a disease state or other undesired condition requiring medical treatment).

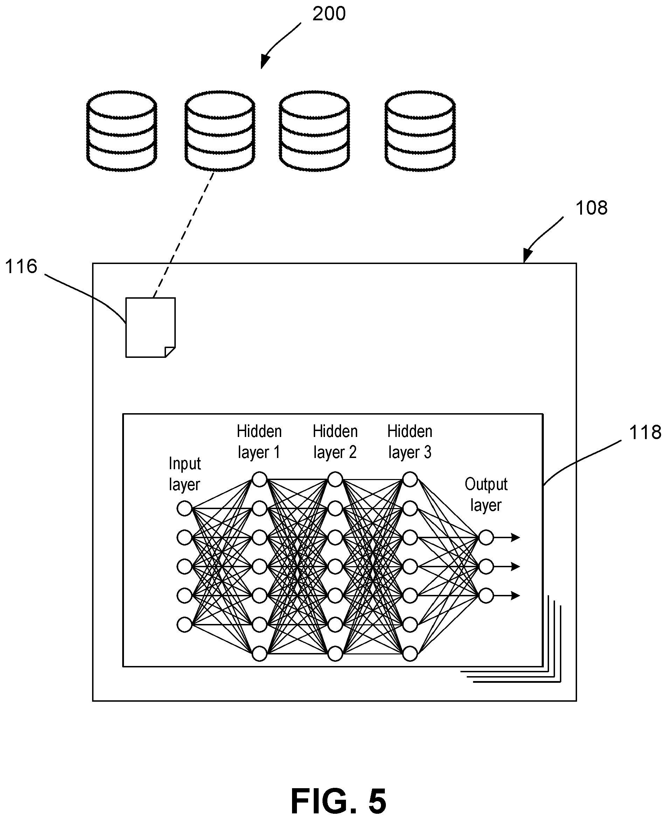

FIG. 5 shows a machine learning system 108 according to certain embodiments of the present disclosure. The machine learning system 108 accesses reference data from the one or more training data sets 116 provided by any known source 200. The source 200 may include, for example, a laboratory-specific repository of reference data collected for purposes of machine learning training. Additionally, or alternatively, the source 200 may include publicly available registries and databases and/or subscription-based data sources.

In preferred embodiments, the plurality of training data sets 116 feed into the machine learning system 108. The machine learning system 108 may include, but is not limited to, a neural network, a random forest, a support vector machine, a Bayesian classifier, a Hidden Markov model, an independent component analysis method, and a clustering method.

For example, the machine learning system 108 an autonomous machine learning system that associates the condition data with the reference ultrasound image data. For example, the machine learning system may include a deep learning neural network that includes an input layer, a plurality of hidden layers, and an output layer. The autonomous machine learning system may represent the training data set using a plurality of features, wherein each feature comprises a feature vector. For example, the autonomous machine learning system may include a convolutional neural network (CNN). In the depicted embodiment, the machine learning system 108 includes a neural network 118.

The machine learning system 108 discovers associations in data from the training data sets. In particular, the machine learning system 107 processes and associates the reference image data and condition data with one another, thereby establishing reference data in which image characteristics of known tissue types and/or anatomical structures are associated with known conditions of the tissue types and/or anatomical structures, including the specific morphology of such tissue types and anatomical structures. The reference data is stored within the reference database 112, for example, and available during subsequent processing of a sample ultrasound image.

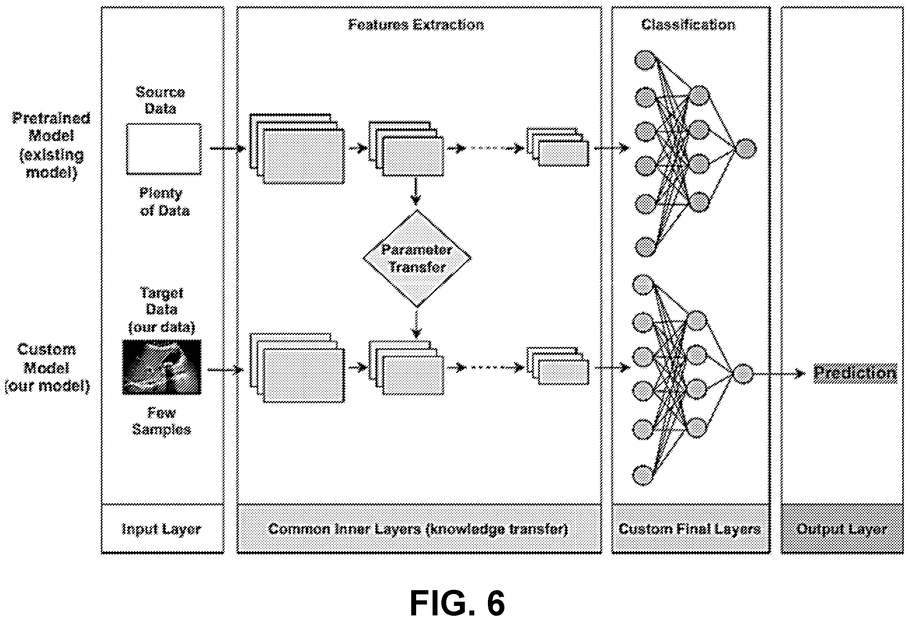

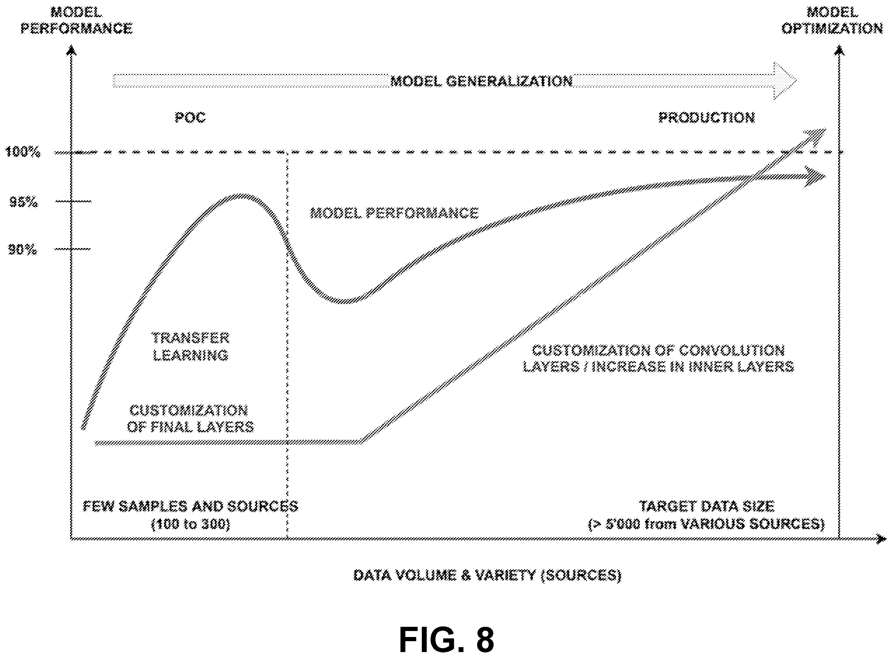

FIG. 6 is a block diagram illustrating one embodiment of a model illustrating transfer learning training of data sets for use in the automated ultrasound image interpretation and analysis consistent with the present disclosure. As shown, such an approach uses transfer learning from established pre-trained models, with the objective to assess the feasibility of the present invention use cases with a small amount of qualified data. It is a common practice to take advantage of the features learned by a model trained on a larger dataset, as illustrated in FIG. 8.

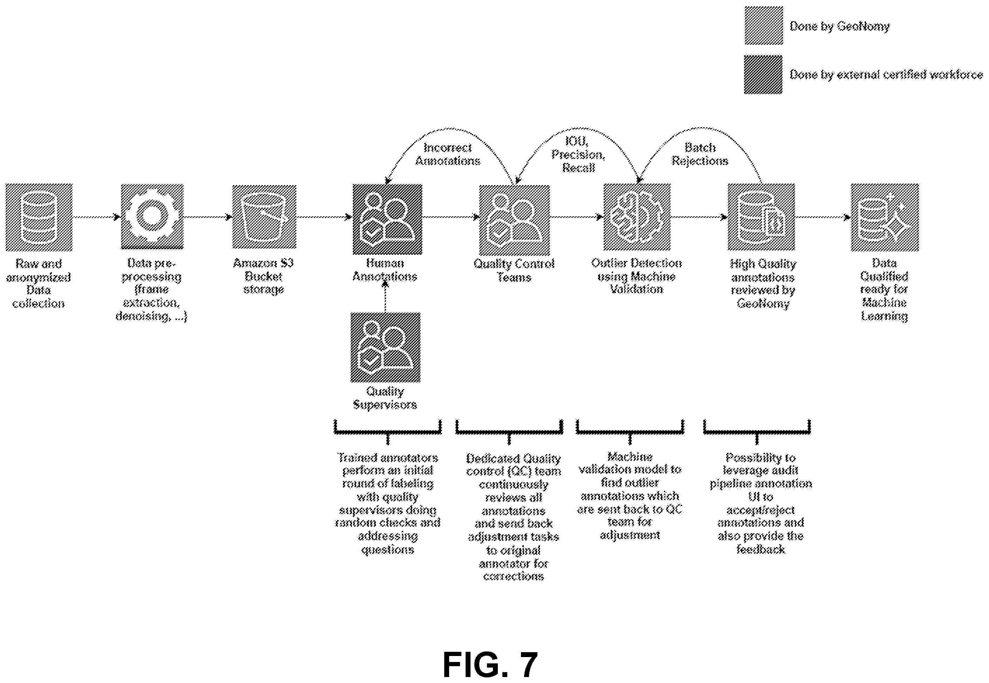

FIG. 7 is a flow diagram illustrating one embodiment of a data qualification and verification process for use with reference ultrasound image data and condition data prior to incorporation into a trained data set of the machine learning system of the present invention. It is apparent that data qualification and verification plays a key role in the development of the machine learning techniques of the present invention. The data qualification and verification process may include use of Amazon SageMaker Ground Truth service. The data engineering pipeline is illustrated in FIG. 7.

FIG. 8 is an exemplary graph illustrating model performance based on the development of training and testing of datasets for use with the machine learning system of the present invention. It is shown that, along with the increase of dataset and data sources to include variability in the training dataset, the machine learning model of the present invention is further customized in the transfer learning, with the objective to achieve the required performance metrics.

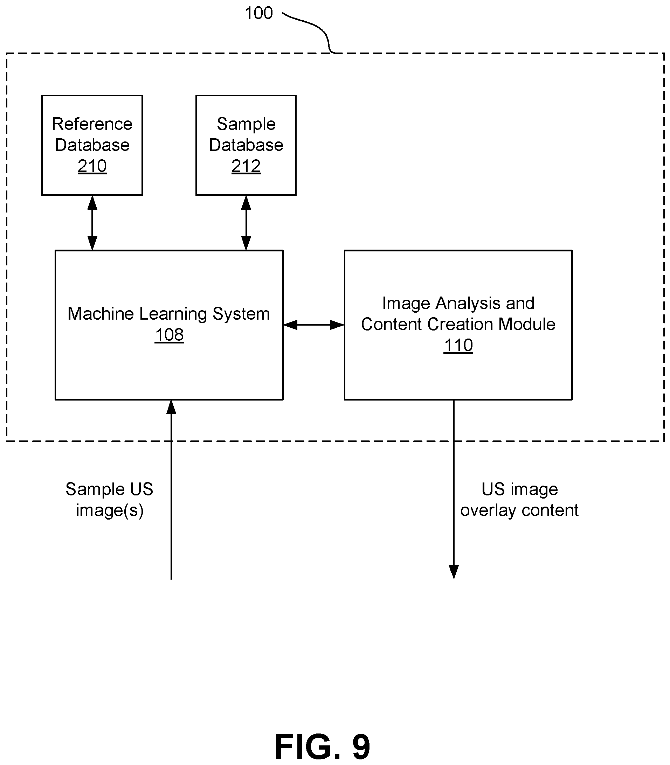

FIG. 9 is a block diagram illustrating receipt of one or more sample ultrasound images acquired and reconstructed during an ultrasound examination of a target site of a patient, subsequent processing of said sample ultrasound images via a machine learning system 108 and image analysis and content creation module of the present disclosure 110, and outputting of ultrasound image overlay content to be displayed in an augmented ultrasound image.

As shown, the system 100 is configured to receive ultrasound image data, which may generally include ultrasound images of a target site of a patient having undergone an ultrasound examination. Upon receiving the sample ultrasound images, the system 100 is configured to analyze the ultrasound images using the neural network of the machine learning system 108 and based on an association of the condition data with the reference ultrasound image data. Based on such analysis, the computing system is able to identify types of tissue and/or anatomical structures within the ultrasound image and further identify a condition associated with any types of tissue and/or anatomical structures identified. More specifically, the machine learning system 108 correlates the sample ultrasound image data with the reference data (i.e., the reference image data and condition data). For example, the machine learning system 108 may include custom, proprietary, known and/or after-developed statistical analysis code (or instruction sets), hardware, and/or firmware that are generally well-defined and operable to receive two or more sets of data and identify, at least to a certain extent, a level of correlation and thereby associate the sets of data with one another based on the level of correlation.

In turn, ultrasound image overlay content can be generated (via an image analysis and content creation module 110). The ultrasound image overlay content is used in providing an augmented ultrasound image, via a display associated with an ultrasound machine 10 and/or the user's computing device 11. The augmented ultrasound image generally includes a visual representation of one or more identified types of tissue and/or anatomical structures at a target site of a patient that has undergone ultrasound image examination, as well any identified abnormal conditions. In particular, the visual representation includes a first layer of content overlaid upon ultrasound images which provides semantic segmentation of different types of tissue and/or anatomical structures (i.e., organs or the like). For example, the first layer may include shaded or patterned zones that are overlaid upon portions of the ultrasound image, as well as associated text indicating each shaded zone represents identified tissue types and/or anatomical structures within the image. The shaded or patterned zones may each have a different color relative to one another to enhance visual distinction of the different tissue and/or structures from one another.

The ultrasound image may further be augmented with a second layer of content that provides an indication of any abnormal condition or anomaly associated with the identified tissues and/or anatomical structures. For example, in the event that the automated analysis identifies an abnormal condition, a second layer is overlaid upon the ultrasound image, which includes a visual indication of the abnormal condition associated with the one or more identified types of tissue and/or identified anatomical structures. The second layer may include, for example, a marking and/or shaded zone corresponding to at least a portion of one of the identified types of tissue and/or identified anatomical structures to which the abnormal condition is associated, as well as text identifying the specific abnormal condition. The abnormal condition may include, for example, a disease or anomaly requiring medical treatment or the like.

The systems and methods of the present invention can be used in the interpretation and subsequent diagnosis of various medical conditions, including, but not limited to, abdominal ultrasound (to visualize abdominal tissues and organs), bone sonometry (to assess bone fragility), breast ultrasound (to visualize breast tissue), doppler fetal heart rate monitors (to listen to the fetal heart beat), doppler ultrasound (to visualize blood flow through a blood vessel, organs, or other structures), echocardiogram (to view the heart), fetal ultrasound (to view the fetus in pregnancy), ultrasound-guided biopsies (to collect a sample of tissue), ophthalmic ultrasound (to visualize ocular structures), and ultrasound-guided needle placement (in blood vessels or other tissues of interest).

For example, one particular use of systems and methods of the present invention include gallstone detection. About 20% of the population of a developed country will, as some point, have gallbladder stones. Its incidence increases with age, female sex, obesity and major changes in weight predominantly. This incidence is therefore expected to increase over the next few years. The presence of gallstones generates a catalog of more or less serious complications all converging on a single therapeutic approach: surgical resection of the gallbladder. In fact, resection of the gallbladder, or a cholecystectomy, is the most commonly performed surgeries in the world.

The systems and methods of the present invention can be particularly useful in providing automatic detection of gallstones, which will allow any clinician to make this diagnosis regardless of their specific level of training and familiarity with reading and interpreting ultrasound images and further, depending on the result, provide necessary medical treatment without wasting time, as it is often the case due to a backlog of cases and time constraints.

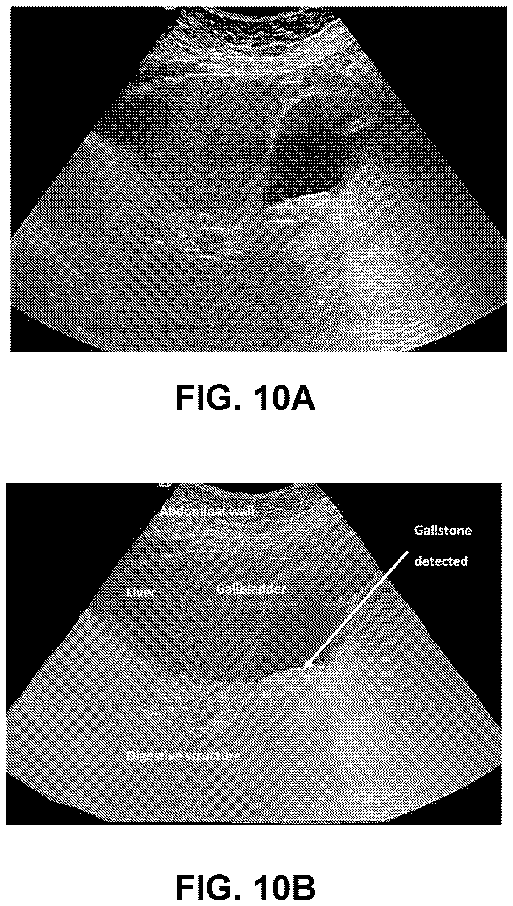

FIG. 10A is an exemplary ultrasound image acquired during an ultrasound examination of a patient's lower abdominal area and FIG. 10B is an exemplary augmented ultrasound image generated based, at least in part, on processing of the ultrasound image of FIG. 10A via the automated ultrasound imaging analysis system, including the machine learning system, consistent with the present disclosure. As illustrated, the augmented ultrasound image of FIG.

10B includes a first layer providing semantic segmentation of the various organs, including segmentation of the liver, gallbladder and digestive structure, as well as a second layer providing an indication of the presence of gallstones in the gallbladder.

The analysis and subsequent generation of the augmented ultrasound image is based on a dataset of at least 150 reference images of gallbladder produced by a radiologist cabinet. These images have been firstly qualified by the specialist and anonymized, then they have gone through an image processing routine to be prepared for machine learning modeling, as previously described herein. In particular, rather than developing reference data sets from scratch, a trained data sets for use in the machine learning system have been created via a transfer learning process from established pre-trained models. The results provide very good performance metrics (94% in sensitivity and 90% in specificity).

Another relevant use of the systems and methods of the present invention include nerve cartography. Locoregional anesthesia accounts for about 20% of all anesthesia in a developed country. This procedure consists of directly blocking a nerve to anesthetize the area that depends on its territory and thus be able to perform operations without completely putting the patient to sleep. Blocking the nerve in practice means injecting an anesthetic product through the skin directly near the nerve without directly injecting into it (which can cause irreversible damage) or injecting into other tissues that are not suitable (such as blood vessels). In the tissues, the nerves rather resemble electric cables which leave the spinal cord to join the tissues for which they are responsible and travel to the midst of other organs along the limbs for example. One of the safest and most effective ways to locate nerves through the skin is via ultrasound guidance. The difficulty with this technique, however, lies in precisely locating the nerves in an ultrasound image during the procedure, which requires additional training and, above all, constant practice to maintain a high degree of awareness.

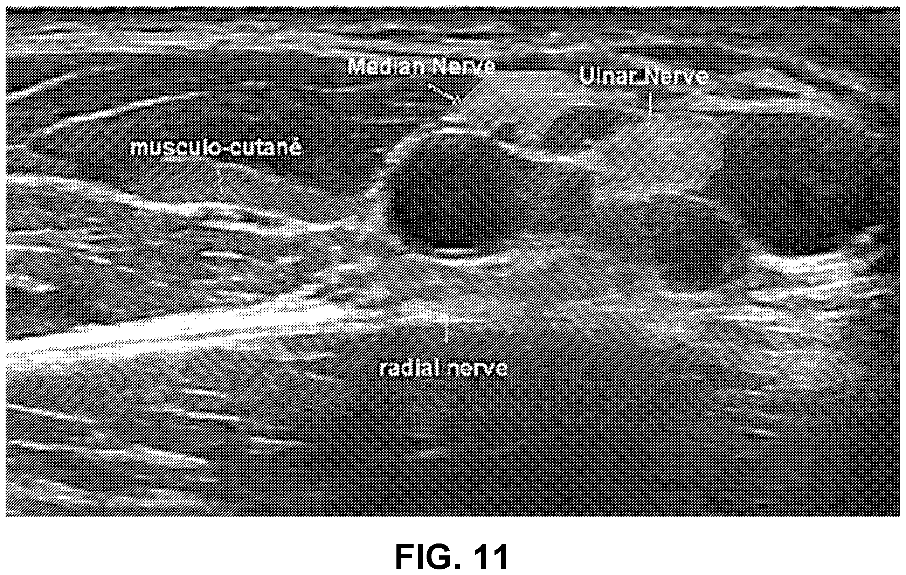

The systems and methods of the present invention can be particularly useful in providing automatic nerves localization and annotation system, by acting in real time. FIG. 11 is an exemplary augmented ultrasound image generated based on the systems and methods of the present invention, which illustrates capabilities of providing automatic nerve localization during an ultrasound examination. The relevant information is superimposed upon an ultrasound image during the procedure, which includes the identified target sites (areas adjacent to target nerve(s)) and the semantic segmentation of the various other anatomical structures contained in the image (muscles, vessels, joints, etc.). This navigation assistance coupled with the automatic identification of the various organs allow the operator to fully concentrate on their main goal of injecting the anesthetic product into the target site, as opposed to attempting to also interpret the image.

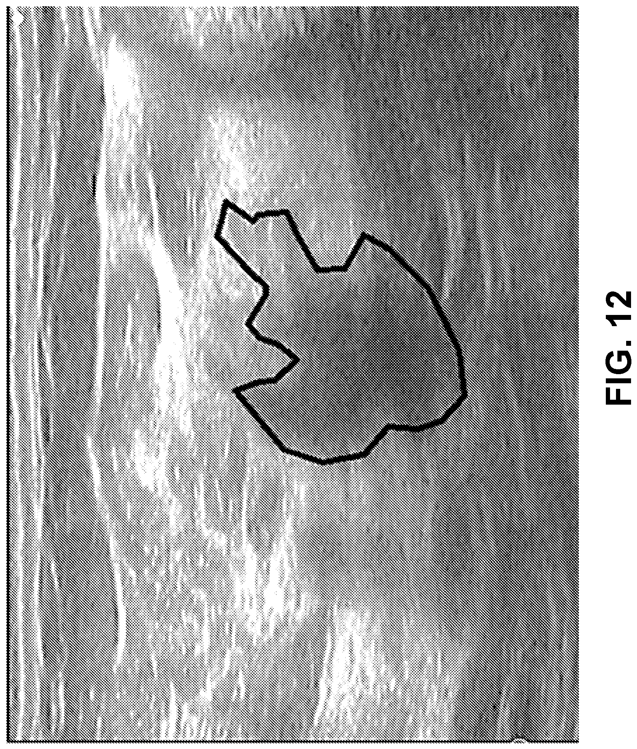

Yet still, another relevant use of the systems and methods of the present invention include cancer detection, including breast cancer detection. FIG. 12, for example, is an exemplary augmented ultrasound image generated based on the systems and methods of the present invention, which illustrates capabilities of providing detection of abnormal growths of tissue (e.g., tumors of the like) within an ultrasound image and an indication of the likelihood that such growth is benign or malignant.

Breast cancer is the number one cancer in the female population and the leading cause of cancer-related deaths. For example, in France, each year there are approximately 54,000 newly diagnosed cases and approximately 12,000 deaths, while in the United States, each year there are approximately 333,000 newly diagnosed cases and approximately 42,000 deaths.

It is known that early detection greatly improves the chances of survival and successful treatment. Accordingly, breast cancer is the first cancer for which systematic screening protocols have been set up at large scale in all developed countries. This screening is based on mammography as an imaging system, supplemented more and more and especially for the youngest women with an ultrasound exam. The purpose of these examinations is to highlight a tumor before it is clinically detectable and to provide information on its degree of mortality. The analysis of the ultrasound image, however, is difficult and, above all, operator dependent. It presents characteristics and subjective nuances to be evaluated by the human eye which, on the other hand, can be interpreted and systematically discriminated by automatic analysis, as provide by the systems and methods of the present invention.

Accordingly, the systems and methods of the present invention can be particularly useful in provided automatic analysis of ultrasound images of breast tissue and provide a determination of whether an identified anomaly can be distinguished between the anomaly of a proven cancerous formation versus all other anomalies or no anomalies at all. This discrimination may require the inference to be done in post-treatment immediately after the ultrasound acquisition.

As previously described herein, the platform of the present invention may either be incorporated directly with an ultrasound machine (i.e., provided as a local component to an ultrasound imaging machine) or may be cloud-based and provide a digital, web-based application that an operator can access via an ultrasound machine or computing device (i.e., smartphone, tablet, personal computer, or the like).

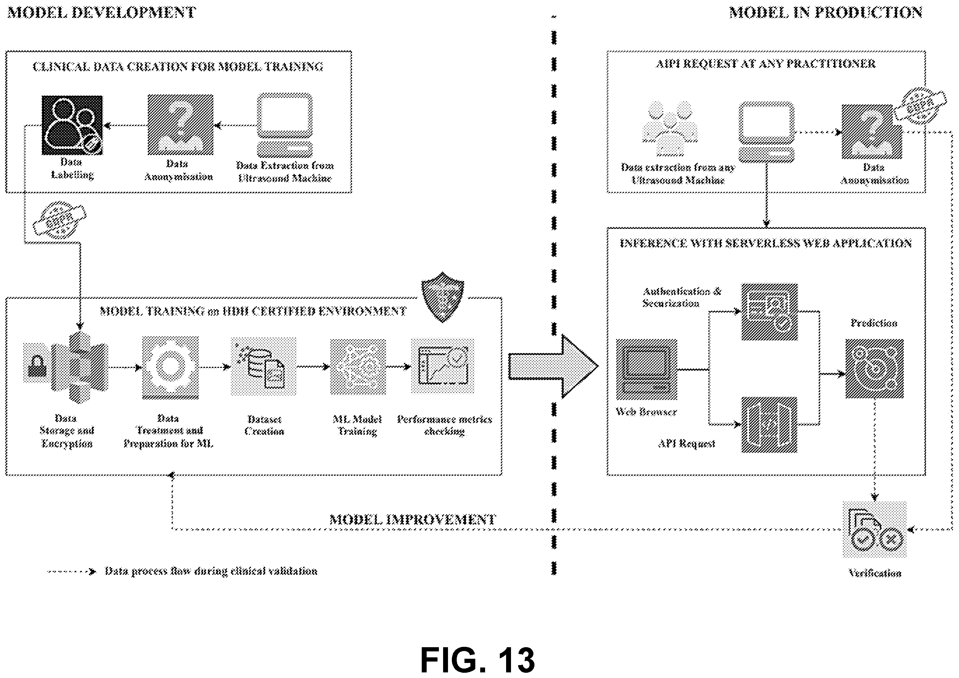

In particular, in one embodiment, the system of the present invention may be provided as an on-demand prediction service. In particular, FIG. 13 is a block diagram illustrating one embodiment of a system consistent with the present invention, which is provided via an on-demand prediction service model. Such a model may be deployed through a web-based application, for example, and can be downloaded onto a personal computing device of a user, such as a laptop, smartphone, and/or tablet.

For example, in such an embodiment in which the platform is cloud-based, such as a software as a service (SaaS) model or the like, the platform is able receive images acquired by any contemplated imaging machine, such as any typical ultrasound imaging machine, and analyze those standard ultrasound images in a deferred manner. In other words, an ultrasound examination may already have been completed, and the ultrasound images from the examination have been saved for later interpretation. Accordingly, an operator need only upload the saved images of interest to the cloud-based platform (i.e., via a computing device, such as a computing device associated with the imaging machine, a smartphone, a tablet, a laptop, or the like). In turn, the platform is configured to analyze the ultrasound images, as previously described herein, and based on that analysis, the platform is able to provide augmented image feedback to the operator, in which the operator may view the augmented ultrasound images on their computing device.

Such an embodiment may generally provide a less expensive near-real time analysis of sample ultrasound images. Furthermore, the availability of CPU power of the associated device is less of an issue since such a model of the system runs on a cloud-based service. Fully managed services, such as AWS, take in charge the infrastructure, the server management, resources availabilities and scaling.

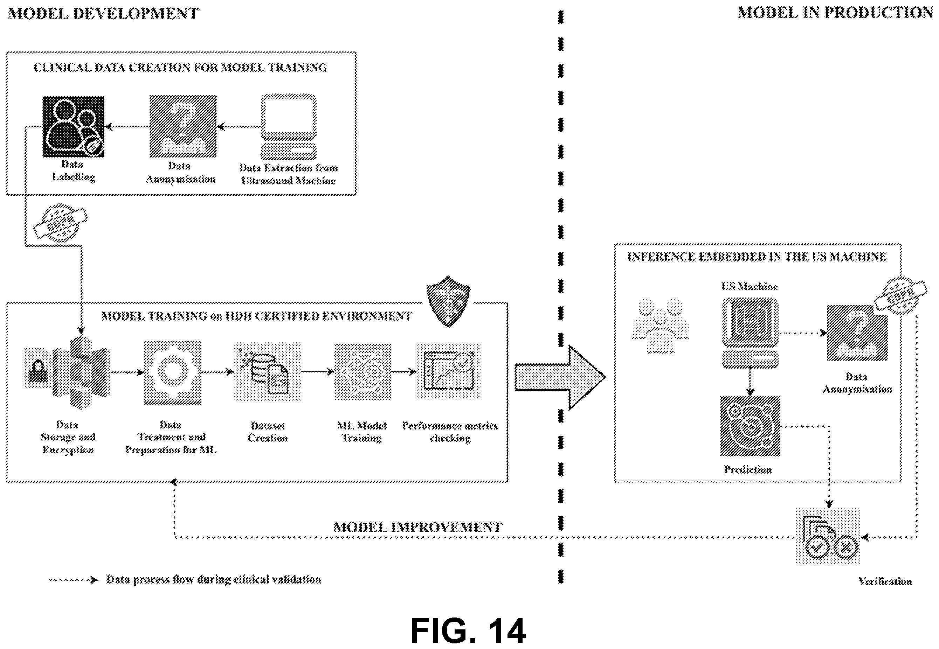

In another embodiment, the system of the present invention may be embedded in an ultrasound imaging machine. FIG. 14 is a block diagram illustrating another embodiment of a system consistent with the present invention, which is provided locally and incorporated into an ultrasound machine. In such an embodiment, the platform is configured to provide real, or near-real, time augmented feedback to an operator of the imaging machine. In particular, the platform is configured to analyze the ultrasound images acquired by the imaging machine during an examination. Based on that analysis, the platform is able to then provide the augmented image feedback to the operator while the examination is occurring, which is generally in the form of an augmented ultrasound image that is presented to the operator in real, or near-real, time during the examination. The main benefits of embedded models is that they can be easily deployed to an ultrasound imaging machine, are able to be customized to the requirements of the machine, and latency is reduced as the machine is likely to be close to the operator than a server away.

Accordingly, by providing automated ultrasound image interpretation and analysis, and further providing augmented image feedback based on artificial intelligence techniques, the present invention addresses the limitations of current ultrasound imaging examinations, namely the constraints associated with interpreting ultrasound images and making a diagnosis. More specifically, unlike a traditional ultrasound examination, which requires the operator to have sufficient knowledge and practice in order to make an accurate diagnosis, the present invention reduces the need for such qualification and training. Rather, any clinician associated with a patient's care and with a minimum amount of training can perform ultrasound imaging and utilize the present invention to receive highly accurate analysis and interpretation of a target site having undergone examination.

Furthermore, by providing the augmented feedback, the present invention provides fundamental information to an operator, including a clear indication of the area of the patient that was imaged, a clear indication and segmentation of various structures captured in the images, and a clear indication of whether such structures are normal or abnormal and require medical intervention. As such, the present invention is able to process ultrasound images based off any standard image, regardless of origin and without modifying the standard ultrasound imaging procedure, thereby improving overall access to ultrasound examinations with a lower level of training and practice, while maintaining high diagnostic efficiency. In particular, the present invention is compatible with most standard and current imaging machines, in that the platform of the present invention analyzes ultrasound images that have already been acquired and reconstructed by the imaging machine and is not required for such image reconstruction.

Anomaly Detection

The systems and methods of the present invention are further configured to determine a level of uncertainty associated with a prediction of a predefined anomaly upon providing automated ultrasound image interpretation and analysis, as previously described herein.

Generally, the ultrasound exam is accomplished in real time by a medical practitioner specialized in ultrasound imagery techniques (e.g., radiologist, gynecologist, etc.) when navigating with an ultrasound probe until an image fit for diagnostic purpose is obtained. Unlike the other medical imaging techniques (i.e., CT scan, MRI, etc.), the ultrasound diagnostic is operator dependent such that the quality of the diagnostic is strongly related to the experience of the operator firstly to localize the anomaly when it is present and secondly to interpret it in a noisy environment. Application of AI techniques, specifically deep learning processing, in the medical imaging field has becoming increasingly popular, from simple image classification to semi-automatic diagnostic work, and many other subjects. However, may neural networks do not produce any measure of uncertainty in their results, which would allow for better qualifying a prediction. Indeed, even though the output of a classical neural network is presented in the form of probabilities, these values do not have a real quality of uncertainty management.

The systems and methods of the present invention are able to exploit the fact that complete information is not contained in a single-shot image, but is rather provided in a succession of images which can be stored in an ultrasound film, in which an average probability accompanied with an uncertainty over the ensemble of image frames can be calculated, with a value of 1 indicating absolute certainty that the patient has the anomaly and a value of 0 indicating absolute certainty that the patient doesn't have the anomaly. The systems and methods of the present invention subsequently allows for the setting up of an uncertainty threshold beyond which the prediction cannot be accepted by the practitioner and requires complementary examination, the threshold being determined during a clinical validation phase.

The systems and methods described herein are configured to produce an exhaustive, automatic and operator-decoupled analysis of an ensemble of consecutive image frames (i.e., up to 300 frames for a film of 10 seconds) rather than focusing on a given moment during the ultrasound examination procedure. The resulting outcome allows modeling a statistical average of the prediction and an uncertainty around the natural variations occurring over the succession of the ultrasound image frames captured along with a pre-defined navigation route of the ultrasound probe on the examined area. The pre-defined navigation route can be part of a protocol imposing to operate the scanning probe from left to right and from top to bottom around the examined area, during a defined time. The resulting natural variations on images are real and translate the different directions of acquisition through different tissue layer compositions, causing an exploitable output variance.

Accordingly, the anomaly detection aspects of the systems and methods of the present invention provide advantages, including supporting the specialist performing an imaging procedure in the establishment of a diagnosis (i.e., in the form of a second opinion for doubtful cases) and allowing for decoupling the creation of the ultrasound images on the patient and their off-line analysis, thus breaking the operator-dependence of the ultrasound examination, and potentially allowing non-specialist of ultrasound to conduct such kind of exam.

As previously noted herein, the systems and method of the present invention can be applied on any kind of pathology which can be identifiable with an ultrasound machine. However, the following description focuses on anomaly detection for the specific case of breast cancer, including the identification of a non-critical situation (absence of a malignant nodule) versus a critical situation (presence of a malignant nodule).

Machine Learning Model Development for Anomaly Detection

In the absence of retrospective ultrasound databases due to the nature of the act which requires the simultaneous presence of the practitioner and the patient, the acquisition of ultrasound images or films is done in a prospective mode (i.e. during a real patient ultrasound examination). As described in greater detail herein, medical data, provided by a practitioner following an examination, is processed.

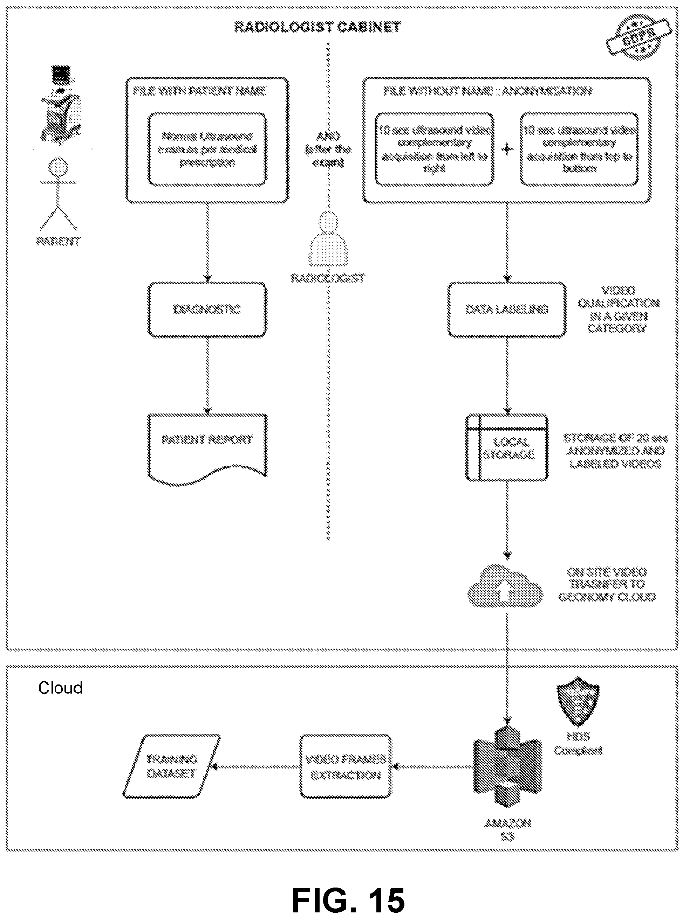

FIG. 15 is a block diagram illustrating one embodiment of a process for collecting and subsequently labeling and validating medical data for developing a machine learning model consistent with the present invention for providing anomaly detection.

Once the specialist (i.e., radiologist, gynecologist, etc.) has completed the prescribed examination on the patient, they close the patient file on the ultrasound machine and immediately opens a new and blank file, repeating exactly the same exam for a short time while registering two consecutive films of 10 seconds each: one film from left to right, another one from top to bottom for each breast. This new file doesn't contain any name or information which could relate it to the patient. The specialist then stores their fully anonymized file in folders on their local computer, each of the folder representing a category including: 1) healthy; 2) adenoma; 3) ganglion; 4) cyst; 5) benign; and 6) malignant. Next, at regular intervals, data is transferred from the local computer at the radiologist cabinet to a secured storage cloud of while being encrypted, such as the automated ultrasound imaging analysis system 100.

It should be noted that the collection of the medical data is secured by several technical and organizational measures which have been defined in order to be compliant with GDPR and public health codes. For example: the anonymization of the data is ensured at the very moment of the examination of the patient by the practitioner (i.e., the ultrasound film is stored without identity nor information about the patient); there is no information provided in the medical data that could allow for the linking of the collected film or image to an identifiable person; the transfer of ultrasound films to the secured storage cloud is done via a secure email or via an encrypted USB key; and only subcontractors who can prove HDS certification are utilized for subsequent handling and processing of the data.

Thus, as the data is anonymized from the medical act carried out by the practitioner, the data processed is no longer considered personal, the processing carried out therefore falls outside the scope of the GDPR and its obligation as to the definition of a retention period of the data. From the point of view of the practitioner acquiring the data for the anomaly detection machine learning model, the act is strictly identical to that provided for by the medical prescription, in that it poses no risk to the patient, it is just repeated once, and quickly (i.e., 20 seconds maximum) and anonymously, so the treatment of the patient is not modified by the collection of the data. It should be noted that the processing of personal data for purposes other than those for which the personal data was initially collected is permitted as it is compatible with the purposes for which the personal data was originally collected. Consequently, the practitioner does not derogate from the Public Health Code.

The data that has been collected and qualified generally comprises ultrasound films with a total duration of 20 seconds maximum each and 30 fps (frames per second). Each US film has been labeled by the professional expert (i.e.,

the radiologist) according to one of the six categories (i.e., 1) healthy; 2) adenoma; 3) ganglion; 4) cyst; 5) benign; and 6) malignant). The machine learning process requires learning from and analyzing individual images corresponding to the frames of the US films. The goal is to use as many appropriate frames extracted from the US films as possible for the machine learning training phase, but, instead of evaluating the model on individual images, the set of images for a given patient are considered. Then, during the model testing or in production, all the frames making the US films are used. For a 20 second US film, this represents a total of 20sec x 30fps = 600 images covering the overall area (the breast) which has been examined. It then becomes statistically possible to determine an average prediction over the 600 images accompanied by a calculation of uncertainty around the natural variations occurring over the succession of the ultrasound frames captured during the ultrasound probe predefined motion on the area of interest.

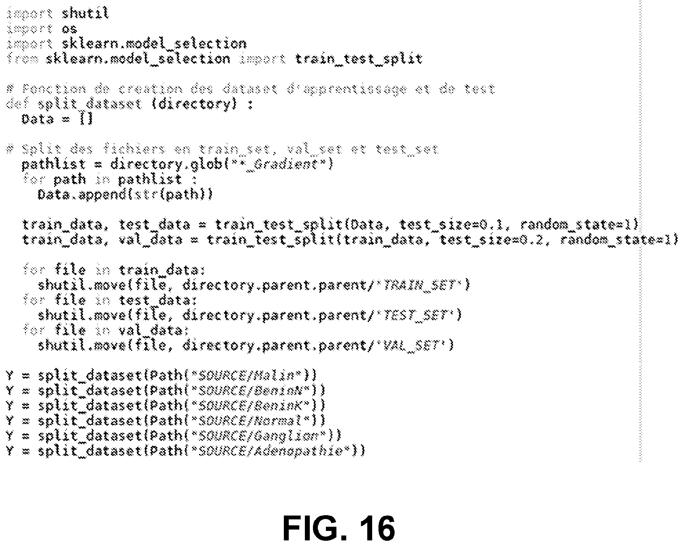

The collected data (i.e., US films) is processed according to rule sets represented by the programming language illustrated in FIGS. 16, 17, and 18.

Referring to FIG. 16, the first function consists of randomly splitting the US films into training, validation and test folders. In particular, the model is evaluated and tested at the level of patient, not at the level of individual images. The training folder contains about 70% of the US films, whereas the validation and test folders contain, respectively, 20% and 10% of the US films.

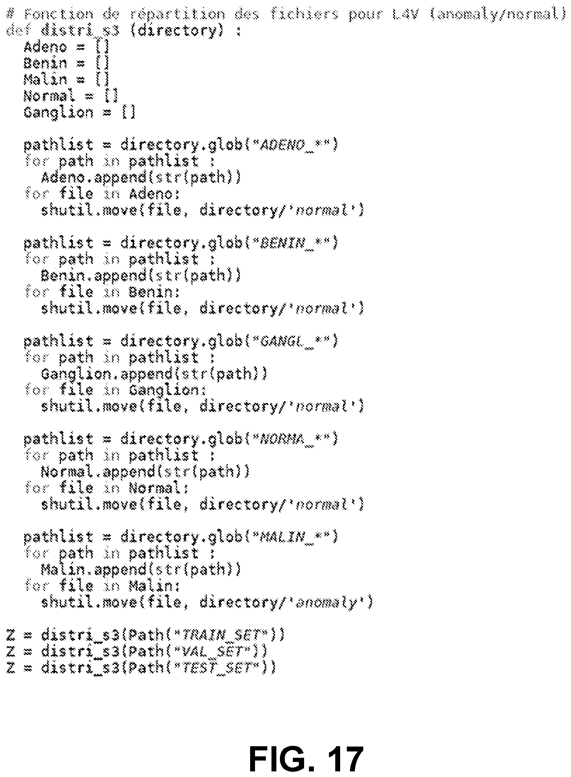

Because the focus is on detecting a critical situation (presence of a malignant nodule) versus a non-critical situation (absence of a malignant nodule and other categories previously noted) a second function (see FIG. 17) consists of regrouping the US films into two classes: anomaly (critical situation) and normal (non-critical situation).

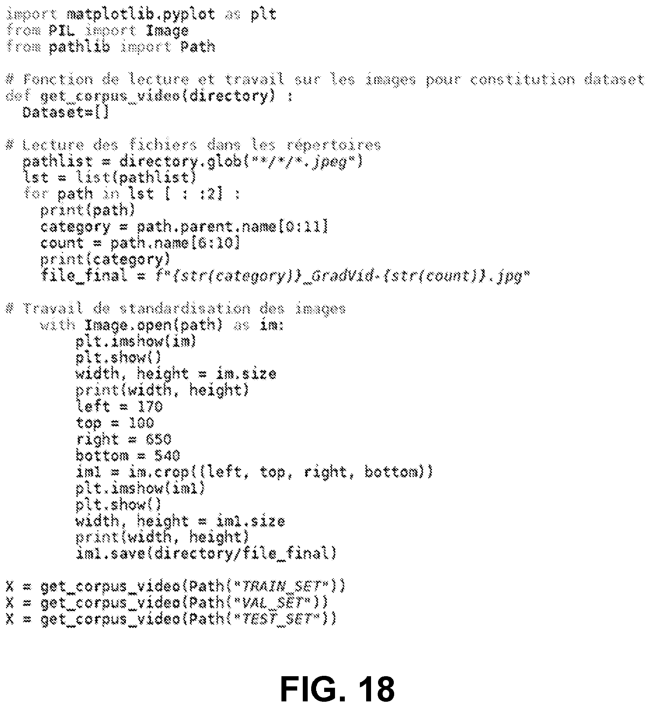

Once the frames have been extracted from the US films of our dataset according to a standard process, a third function (see FIG. 18) is used to prepare the final training, validation and test dataset composed of frames extracted from the US films.

The present invention recognizes that it is desirable to not have very similar consecutive images in a given training dataset, as it could alter the learning process. Accordingly, the present invention may use of a sampling ratio in order to retain appropriate images (i.e. the minimum interval in time allowing a visual difference between successive images). For example, in one embodiment, a sampling interval of 2 seconds, corresponding to a sampling ratio of 1/60, has been applied on all the frames of the training dataset only. Then, the image size has been standardized for an optimized ingestion in the machine learning model, and have been named in order to keep a traceability.

The overall dataset contains 1,421 images in jpeg format, from 127 ultrasound films of 20 second each on average, recorded by a radiologist expert in senology on a panel of 127 all-around patients, and is split into a training dataset, a validation dataset, and a test dataset. The training dataset contains 983 sampled images according to the explanation above, distributed into 326 anomaly classified images and 657 normal classified images. It is used to fit the parameters to the machine learning model. The validation dataset contains 279 images belonging to 24 US films of 24 all-around patients with 6 anomaly classified films and 18 normal classified films. It is used to provide an unbiased evaluation of the model fitted on the training dataset while tuning model hyperparameters. The test dataset contains all images extracted from 15 US films of 15 all-around patients with 4 anomaly classified films and 11 normal classified films. It is used to provide an unbiased evaluation of the final model fitted on the training dataset.

The present invention includes an image classification model, that allows each image (frame) of the same film to be associated with one of the two predefined classes: 1) anomaly (critical); or 2) normal (non-critical). As such, it is binary classification problem. In order to develop the image classification model, an anomaly detection model has been used, the anomaly detection model having been trained to recognize a non-critical situation (normal class, regrouping everything except a malignant nodule) versus a critical situation (anomaly class for malignant nodule only).

The evaluation of the model is made on the validation dataset and leads to the results summarized in the table 1 below.

| Table 1: Results of Model Evaluation | | |
|---|---|---|
| | **At image level** | **At patient level** |
| **Population** | 279 images | 24 US films |
| **True Negative** | 60 | 5 |
| **False Negative** | 16 | 0 |
| **True Positive** | 175 | 18 |
| **False Positive** | 28 | 1 |
| **Sensitivity** | 92% | 100% |
| **Specificity** | 68% | 83% |

The evaluation consists of comparing the model predictions versus the ground truth (i.e., the label associated by the practitioner to the US film during the data acquisition and qualification phase).

The observations get a predicted score in the range [0,1]. The threshold score for deciding whether to classify examples as 0 (normal class) or 1 (anomaly class) is set by default to 0.5 (or 50% prediction). The "At image level", the True Negative, False Negative, True Positive, False Positive are determined based on the individual prediction score of each class which is higher than 0.5 for all the 279 images of the validation dataset. The "At patient level", the True Negative, False Negative, True Positive, False Positive are determined based on the average prediction score of each class which is higher than 0.5 over the entire set of images of a given US film.

Since the invention is related to a medical diagnostic, the most important result lies in the specificity parameter which describes here the probability of diagnosing an anomaly when it is really present. Indeed the primary objective of the practitioner for such a use case is to not miss a critical situation. As such, the evaluation results in table 1 above show that the specificity is significantly improved when inferring all the frames of a US film and averaging the results for each class.

Once the model has been evaluated and its optimization process completed, it is tested on a separate dataset kept aside, never used during the training and evaluation phase, in order to provide an unbiased evaluation of the final model after optimization.

The test of the model is made by inferring the model for all the frames of a US film belonging to the test dataset and comparing the average prediction over the full set of images versus the ground truth (i.e., the label given by the practitioner to the US film during the data acquisition process).

For a 20 second US film composed of 30 fps, the model can be inferred over N images with Nmax = 600. Each image is analyzed and a prediction score $Pred_{i\_k} \in [0, 1]$ with $i \in [1,N]$ and $k \in [1,2]$ is given for each k class (Anomaly or Normal).

Once all the N images have been analyzed through the model, an average score for each class $\mu_{pred\_k}$ is calculated and it can be determined the prediction class as the class having the highest average score. Then, the selected class versus the ground truth label are compared. The standard deviation over the N images and for each class ($\sigma_{pred\_k}$) is further calculated. The standard deviation is generally a good parameter to assess the uncertainty, this later being a key factor allowing to indicate how much confident we are from the model prediction. The uncertainty represents the degree of belief for the practitioner. It is evaluated versus an uncertainty threshold which is determined during a clinical study.

The tables below (tables 2 and 3) illustrate the results with the test dataset, in which a total of 151 images from the 15 US film have been extracted and compose the test dataset.

| Table 2: Contingency Table at Image Level | | |
|---|---|---|
| | Prediction_Anomaly | Prediction_Normal |
| Groundtruth_Anomaly | 76 | 20 |
| Groundtruth_Normal | 2 | 53 |

| Table 3: Results of Model Testing | | |
|---|---|---|
| | At image level | At patient level |
| Population | 151 images | 15 US films |
| True Negative | 53 | 4 |
| False Negative | 20 | 3 |
| True Positive | 76 | 8 |
| False Positive | 2 | 0 |
| Sensitivity | 79% | 73% |
| Specificity | 96% | 100% |

The test results in table 3 above show that the specificity is maximized when inferring all the frames of a US film and averaging the results for each class.

Validation of Anomaly Detection Machine Learning Model via Clinical Study

Once the model has been tested and its optimization completed, it is validated through a clinical study carried out to assess the efficacy of the anomaly detection method, compared to a human reference diagnostic.

One outcome of the clinical study is the determination of an uncertainty threshold ($\sigma_{thre}$) over which the associated model prediction is not acceptable, thus requiring a complementary examination. In this way, when $\sigma_{pred} > \sigma_{thre}$ the model prediction cannot be taken into consideration, when $\sigma_{pred} < \sigma_{thre}$ the model prediction is acceptable.

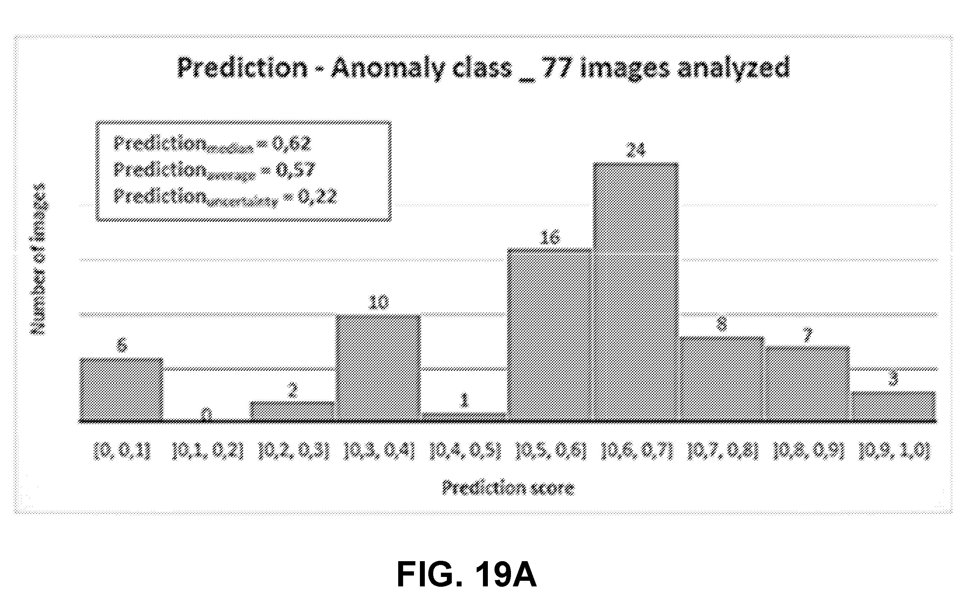

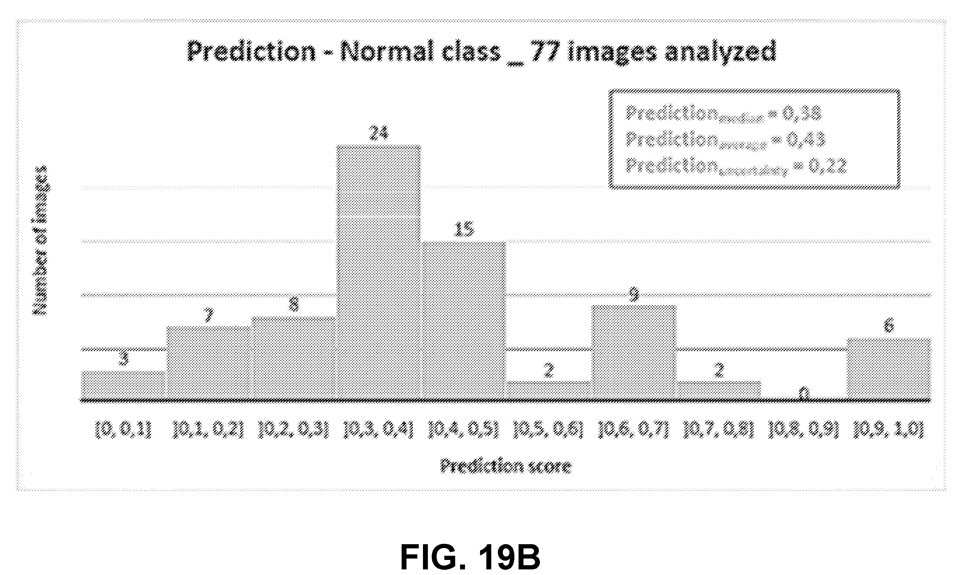

FIGS. 19A and 19B are bar graphs illustrating prediction results and analysis of anomaly classification and normal classification, respectively, consistent with the systems and methods of the present invention. As shown, the bar graphs are based on a set of 77 consecutive images of a given US film which presents a ground truth label = Anomaly. The results show that the Anomaly class obtains the best results in terms of average prediction score over the 77 images ($\mu_{pred\_anomaly} = 0{,}57 > \mu_{pred\_normal} = 0{,}43$) and in terms of median prediction which represents the midpoint of the image set predictions (median$_{pred\_anomaly} = 0{,}62 >$ median$_{pred\_normal} = 0{,}38$). This 2 parameters suggest a diagnostic in favor of an anomaly.

However, it should be noted that the individual image predictions are widely spread, with an important standard deviation for both classes ($\sigma_{pred\_anomaly} = \sigma_{pred\_normal} = 0{,}22$) which naturally affect the uncertainty. If during the clinical study, the uncertainty threshold has been defined at $\sigma_{thre} = 0{,}1$, then the model prediction above is notified as NON acceptable or Rejected, requiring complementary exams for the patient.

Deployment of Anomaly Detection Machine Learning Model

Once the anomaly detection machine learning model has been validated, it is integrated with the pre-processing and post-processing modules. The pre-processing module may include the US film ingestion and the preparation of the data which will be analyzed by the model, while the post-processing module may generally include the computation of the average prediction scores and uncertainties over the full set of images making the US film, the selection of the predicted class and its acceptability.

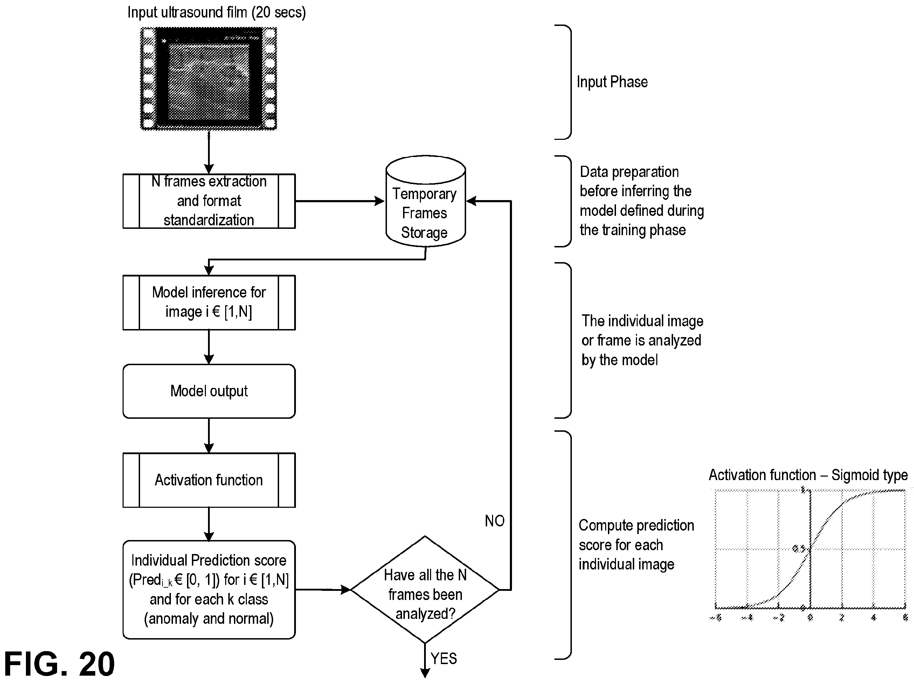

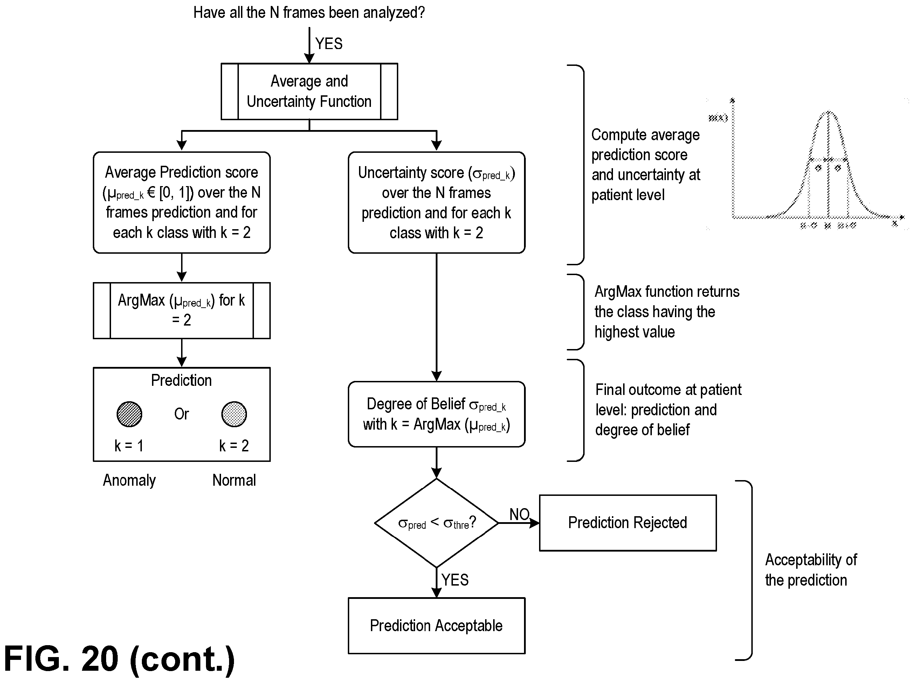

FIG. 20 illustrates the overall workflow of an anomaly detection framework of the present invention. The overall workflow is generally "hosted" in a web application (i.e., "Web App" or "App"), downloadable from any browser, which allows the practitioner, using secure access details, to infer the model by downloading their own ultrasound films.

For example, a user (i.e., practitioner) can utilize the web app (via a computing device) to access an anomaly detection machine learning model consistent with the present disclosure. The user can interact with an interface allowing for user input, particularly allowing for a user to provide login credentials (i.e., username, email address, or other identifying information, and a password). In turn, the user may then access a specific model available for inference.

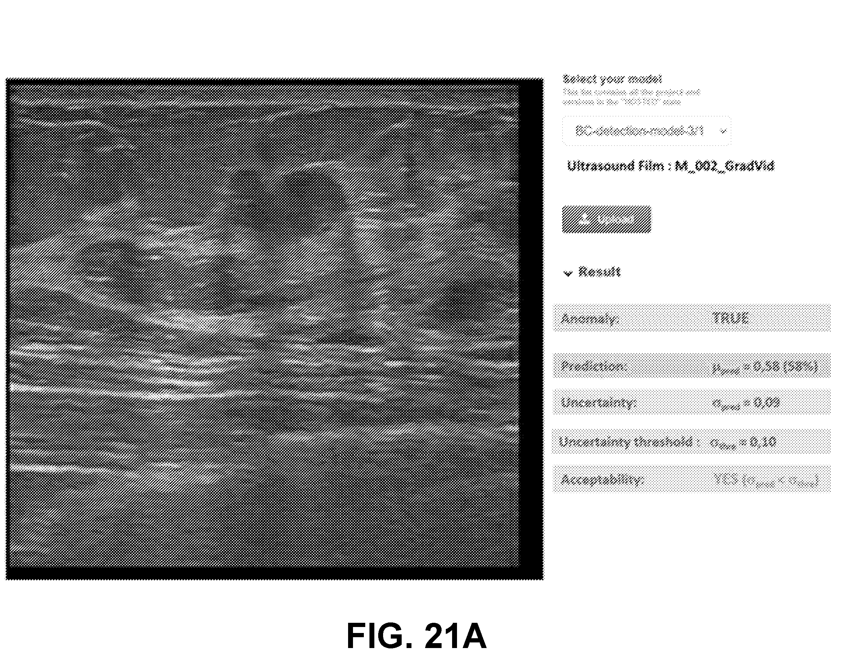

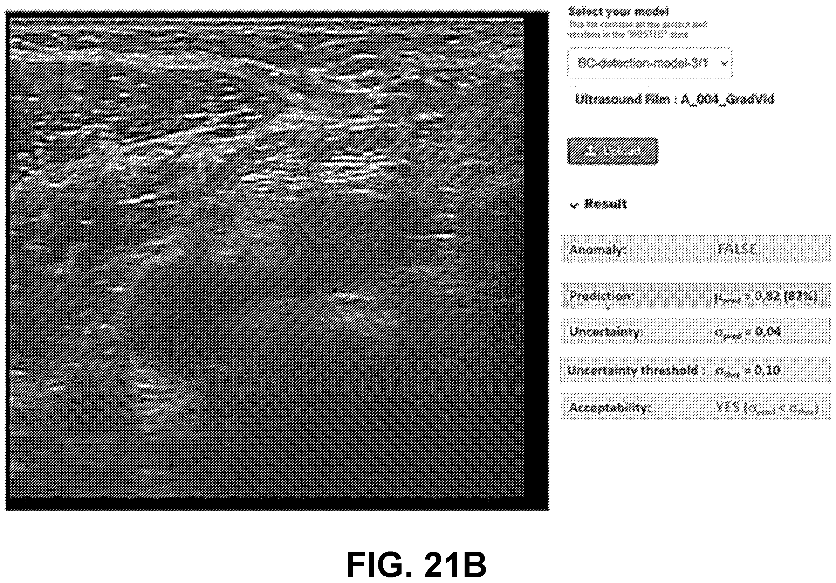

Once a model version has been selected, it is possible to import a US film to detect if the patient presents an anomaly (TRUE/FALSE). In turn, the algorithm automatically processes the US film in the "background" to extract the frames which will successively infer the model. Then the algorithm calculates an average prediction by class, based on which the detection result is provided (TRUE for anomaly or FALSE for normal), and the associated uncertainty over the entire set of frames making the US film. In function of the uncertainty value calculated, the application indicates whether the result can be accepted ($\sigma_{pred} < \sigma_{thre}$) or rejected ($\sigma_{pred} > \sigma_{thre}$). FIGS. 21A and 21B are screenshots of an exemplary user interface of the web app, illustrating a sample US film provided by the user and subsequent determination of various characteristics of the film based on processing/analysis performed via the anomaly detection machine learning model consistent with the present disclosure.

Validity of Averaging Individual Predictions

The averaged prediction calculated over the set of images making the US films is based on the arithmetic average of class probabilities predicted for each image. Indeed, for this problem of binary classification where the model must choose between only two labels, we use the Sigmoid function as the activation function. The Sigmoid function is applied at the output of the artificial neurons and provides a value between 0 and 1, which can be seen as a probability.

From the law of total probability we know that for disjoint events Image$_n$ where $n \in [1,N]$ and N the total number of consecutive images analyzed by the model, we can calculate the probability P for both class "Anomaly" and "Normal" :

$$P(Anomaly) = \sum_{n=1}^{N} P(Anomaly|Image_n) \; x \; P(Image_n)$$

$$P(Normal) = \sum_{n=1}^{N} P(Normal|Image_n) \; x \; P(Image_n)$$

Basically if $P$(Anomaly|Image$_n$) with $n$:1,...,$N$ are different images emitting probabilities, and Image$_n$ is a disjoint hypothesis space, then the result is a probability.

When doing simple averaging we are assuming equal probability to each of the N images, i.e. $P$(Image$_n$) = 1/$N$ for all $n$: 1,..,$N$; a discrete uniform distribution.

As previously discussed in the above "Validation of Anomaly Detection Machine Learning Model via Clinical Study" section, we have used 77 consecutive images from a given US film to calculate the averaged prediction which can be expressed as follow :

$$P(Anomaly) = \ P(Anomaly|Image_1)x\ P(Image_1)$$
$$+ P(Anomaly|Image_2)xP(Image_2)$$
$$+ P(Anomaly|Image_3)xP(Image_3)$$
$$+ \cdots$$
$$+ P(Anomaly|Image_{77})xP(Image_{77})$$

Knowing that all the consecutive Images of a US film can be considered as disjoint hypothesis, we can set up the following:

$$P(Image_1) = \ P(Image_2) = \ P(Image_3) = \cdots \ = P(Image_{77}) = 1/77$$

As a consequence,

$$P(Anomaly) = \frac{1}{77}\ x \sum_{n=1}^{77} P(Anomaly|Image_n)$$

$$P(Normal) = \frac{1}{77}\ x \sum_{n=1}^{77} P(Normal|Image_n)$$

Accordingly, the anomaly detection provided by systems and methods of the present invention contributes to the acceptability of AI in the medical field by proposing an uncertainty (degree of belief) accompanying the prediction of a pre-defined anomaly or not. For that it is exploiting the full set of images making an US film when inferring an already optimized machine learning model. The present invention can further be improved with the implementation of deep learning Bayesian technics, which introduce a probabilistic approach also during the machine learning model development phase.

Detection and Tracking Tissue Characteristics (in Real Time)

The systems and methods of the present invention are further configured to train a statistical model to automatically describe tissue and organs encountered during an ultrasound examination. In particular, as described in greater detail herein, the process of training the statistical model includes collecting medical data and determining qualification of such data via a system of annotation of the organs on the image frames making the ultrasound films. The statistical model is trained with a set of image frames associated with a corresponding patch annotation determined based on a subset of pixels (semantic segmentation).

The following description focuses on detection and tracking tissue characteristics for the specific use related to anatomical topography via a semantic segmentation, specifically the automatic detection of axillary nerves for upper limb operations without general anesthesia, referred to herein as "nerve cartography".

As previously described herein, a relevant use of the systems and methods of the present invention includes nerve cartography. Locoregional anesthesia accounts for about 20% of all anesthesia in a developed country. This procedure consists of directly blocking a nerve to anesthetize the area that depends on its territory and thus be able to perform operations without completely putting the patient to sleep. Blocking the nerve in practice means injecting an anesthetic product through the skin directly near the nerve without directly injecting into it (which can cause irreversible damage) or injecting into other tissues that are not suitable (such as blood vessels). In the tissues, the nerves rather resemble electric cables which leave the spinal cord to join the tissues for which they are responsible and travel to the midst of other organs along the limbs for example. One of the safest and most effective ways to locate nerves through the skin is via ultrasound guidance. The difficulty with this technique, however, lies in precisely locating the nerves in an ultrasound image during the procedure, which requires additional training and, above all, constant practice to maintain a high degree of awareness.

The systems and methods of the present invention can be particularly useful in providing automatic nerves localization and annotation system, by acting in real time. Referring back to FIG. 11, an exemplary augmented ultrasound image is generated based on the systems and methods of the present invention, which illustrates capabilities of providing automatic nerve localization during an ultrasound examination. The relevant information is superimposed upon an ultrasound image during the procedure, which includes the identified target sites (areas adjacent to target nerve(s)) and the semantic segmentation of the various other anatomical structures contained in the image (muscles, vessels, joints, etc.). This navigation assistance coupled with the automatic identification of the various organs allow the operator to fully concentrate on their main goal of injecting the anesthetic product into the target site, as opposed to attempting to also interpret the image.

The acquisition of prospective ultrasound films is conducted by medical specialists during a real intervention on real patients but anonymously and without modifying the normal examination procedure, thus making the data collection process compliant with the law. The use case "nerve cartography" represents a situation where the patient is having an intervention on the upper limbs, requiring a transcutaneous anesthesia of the brachial plexus, generally conducted by the anesthesiologists.

Brachial plexus is a complex network of nerve roots that coalesce into proximal trunks, then divide into cords and distal branches, from the neck to axilla. At the junction of the axilla and upper arm, peripheral nerves arise from the brachial plexus, to supply both the sensory and the motor innervation of the upper limb. An accurate description of the brachial plexus anatomy at this site is of great importance. Ultrasound-guided techniques are generally used to achieve the level of accuracy required.

The ultrasound probe is placed perpendicular to the skin of the axilla, at the intersection of the pectoralis major muscle with the biceps brachii. The probe is applied, with light pressure, just enough to collapse the main veins surrounding the axillary artery, without changing anatomic structures. The ultrasound beam is set perpendicular to the brachial plexus nerves and the axillary artery, so that they appeared in short axis as round or oval structures on the ultrasound scan. Four nerves (radial, ulnar, median, and musculocutaneous nerves) are then located during this identification phase. After locating each nerve, their blocking is performed using in-plane technique by slowly injecting local anesthetic solution around the nerves. Needle position is adjusted to provide circumferential spread of local anesthetic around each nerve. During the injection phase, the anesthetist is using one of his hand to control the ultrasound probe and the other hand to operate the needle while an assistant is setting the local anesthetic dose to inject. The same operation is repeated for the different nerves to anesthetize.

The statistical model of the present invention generally includes: 1) automatically identifying the four nerves; 2) determining their shape with semantic segmentation technics, in real time, on the ultrasound film that the anesthetist is visualizing; and 3) tracking the nerves through the overall anesthesia process from the identification phase to the injection of the anesthetic solution around the nerves.

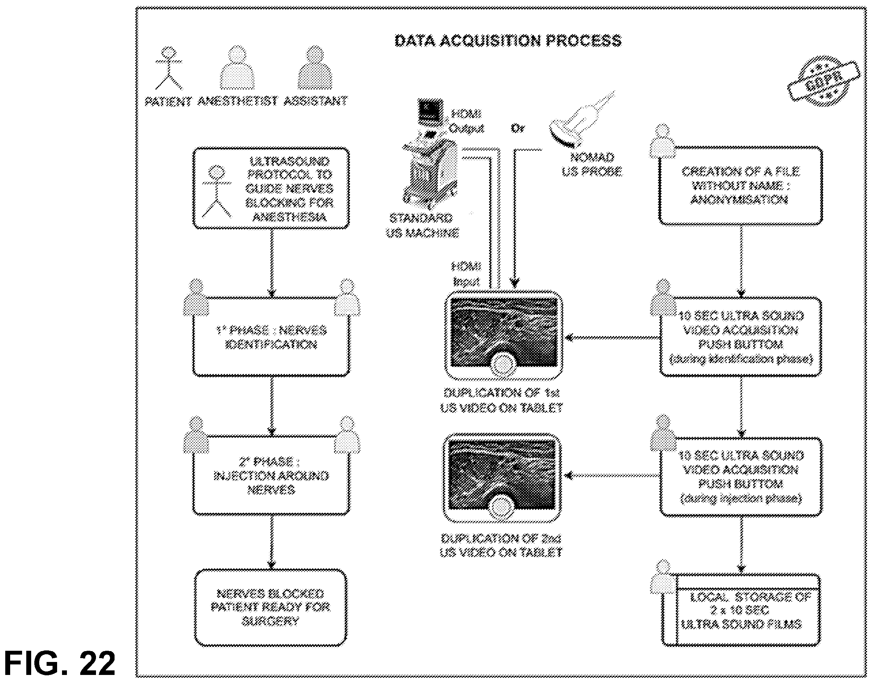

FIG. 22 shows a flow diagram illustrating the process of collecting the required data for training a statistical model to automatically describe tissue and organs encountered during an ultrasound examination, specifically for anatomical topography via a semantic segmentation, including the automatic detection of axillary nerves for upper limb operations without general anesthesia, referred to as "nerve cartography". As generally understood, a personal computing device, such as a PC, tablet or smartphone may be ergonomically settled in the physical environment around the anesthetist and his assistant. The device may either be directly connected to an ultrasound imaging (nomad) probe or to a standard ultrasound imaging machine via an HDMI connection (or other wired connection), such that its transformation into a local internet signal and the use of the internet signal by ethernet to the computing device.

The collection of ultrasound (US) film is secured by several technical and organizational measures which have been defined in order to be compliant with GDPR and public health codes. For example, the anonymization of the data is ensured at the very moment of the examination of the patient by the anesthetist: the ultrasound film is stored without identity nor information about the patient; no information is provided that could allow to link the collected film or image to an identifiable person; the acquisition act is identical to that provided for the purpose of nerves anesthesia (as it poses no risk to the patient, it is just repeated once, quickly (10 seconds per acquisition), and anonymously, so the treatment of the patient is not modified by the collection of the data; the transfer of ultrasound films is done via a secure email or via an encrypted USB key, directly collected by key team members and uploaded to a secured storage cloud of while being encrypted, such as the automated ultrasound imaging analysis system 100; and only subcontractors who can prove HDS certification are utilized for subsequent handling and processing of the data.

## Manual Data Labeling

The data qualification is made by a group of anesthesiologists (professional experts) on the image frames extracted from US video acquired during the collection process. The objective is to super-impose on the images an annotation describing a tissue characteristic category for a portion of the individual image frame.

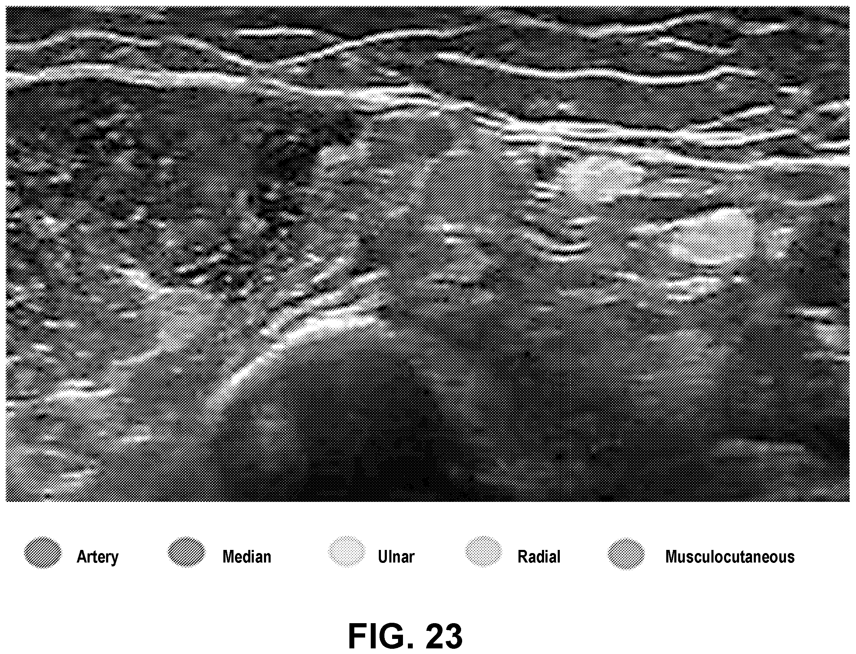

The images may be annotated with polygon objects that draw the outlines of different organic structures: artery, median nerve, ulnar nerve, radial nerve and musculocutaneous nerve. Additional image processing may be carried out to build the training dataset that can be used by the machine learning models. In particular, the systems and methods of the present invention may utilize a script that has been developed to convert polygon annotation objects into fully colored segmentation masks, as illustrated in FIG. 23, which shows segmentation of different organic structures visually indicated via different colored segmentation masks.

## Model Learning and Evaluation

The systems and methods of the present invention utilize a semantic segmentation model, which allows for each image (i.e., frame) of the same film to be associated with a corresponding patch annotation determined based on a subset of pixels.

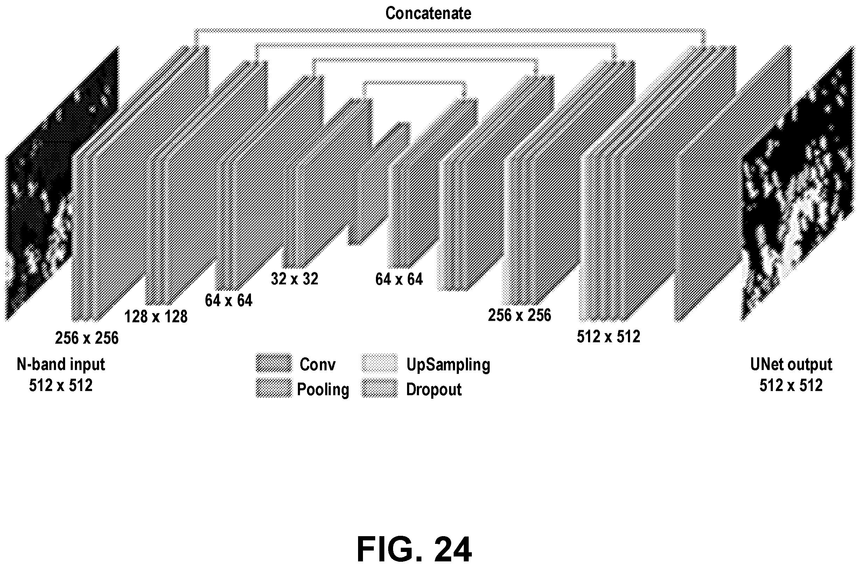

For example, an initial learning dataset may include 764 annotated images in jpeg format, from 29 ultrasound films, made by a group of anesthesiologists (professional experts) on a panel of 29 all-around patients. The learning phase may consist of calibrating an algorithm which learns, as a result of processing to annotated images, to classify each pixel of an image in one of the predefined classes (the nerves). The architecture selected is a U-net++ type auto-encoder, as shown in FIG. 24, in which the architecture is a serial coupling of 2 convolutional neural networks: 1) the first block "encodes" the pixels of the image until obtaining a vector of pixels which associates each pixel with a class; and 2) the second block "decodes" the vector of pixels in order to replace them in the space to recreate a raster image with a color for each pixel corresponding to each learning class.

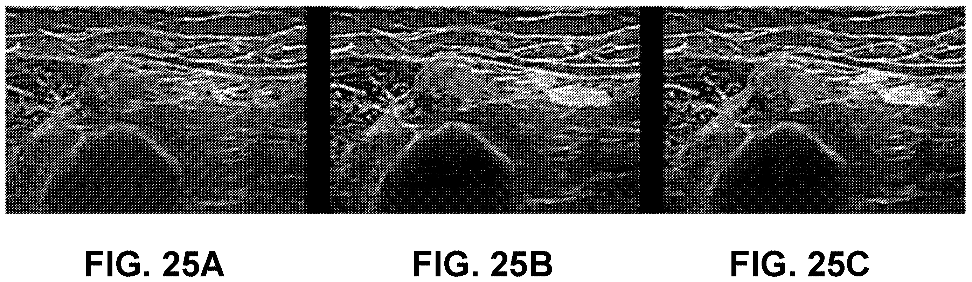

The evaluation of the semantic segmentation model consists of running the model on the films of the validation dataset, which did not participate in the learning phase: each image of a film of the dataset is analyzed through the auto-encoder; the image is transformed into a vector of pixels with which a given class of tissue/organ is associated; then the image is reconstructed but with colors corresponding to each class producing a colorized mask (shown in FIGS. 25A, 25B, and 25C). In particular, FIG. 25A is an image of the original film, FIG. 25B is a prediction given by the model (colorized mask for each type of class), and FIG. 25C is the ground truth with the segmented classes following the annotation made by the professionals.

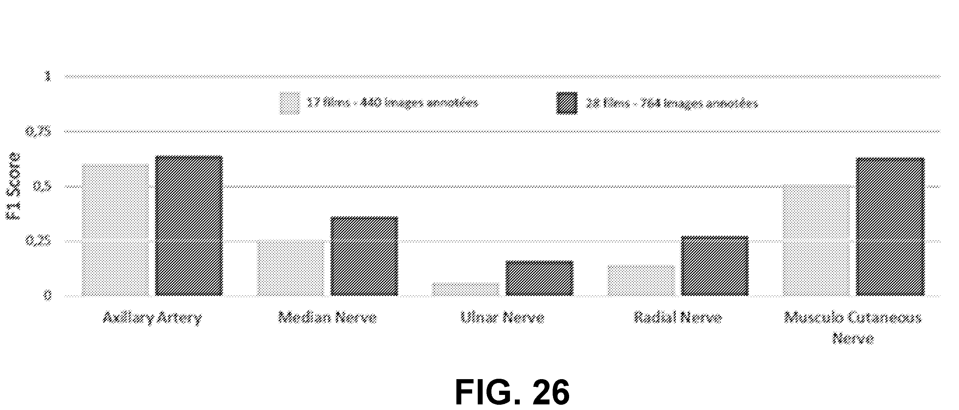

The performance of the model on the training dataset can be expressed by the F1-score, a global performance indicator calculated on all the successive frames of an ultrasound film and taking into consideration the class prediction of each pixel with respect to the ground truth. This indicator varies from 0 to 1 where 1 corresponds to a maximum performance of 100%, as shown in the bar graph of FIG. 26. As shown, the model works well on 2 of the 5 classes to be identified (overall performance > 50%). The other 3 classes require more data to stabilize the model. This is highlighted by the comparison of the graph above: the addition of 324 images from only 11 additional patients significantly increased the performance of the model on the median and musculocutaneous nerves and doubled the performance on the radial and ulnar nerves. The model benefits greatly from new data, which is continually acquired over time with the support of anesthesiologists.

## Post-Processing

While semantic segmentation allows for detecting the nerves and representing them by a mask, a practical problem concerning injection of a local anesthetic solution around the nerves may still exist for the anesthetist (i.e., it is crucial for them to clearly see the nerves boundaries before injecting). Even when the performance of the model gets excellent, the masks may disturb the anesthetists by preventing them from having a clear view of the location where to make the injection.

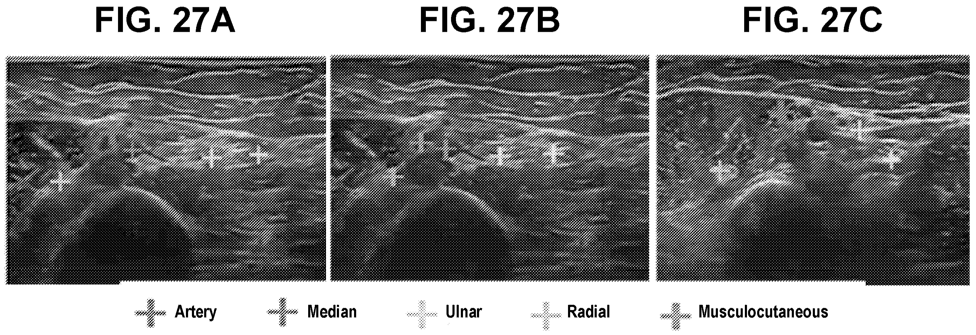

In an effort to overcome this problem, the systems and methods of the present invention further include a means for calculating the barycenter of the colorized masks representing the nerves, removing them and super-imposing a cross onto the barycenter. Moreover, and because during the motion of the probe against the skin the anesthetists may "lose" some nerves (typically the radial and ulnar nerves), the present invention further includes tracking techniques which combine spatial and temporal tracking information by fixing the different crosses and following them as soon as they have been settled. For example, FIGS 27A, 27B, and 27C are ultrasound images in which the artery, median nerve, ulnar nerve, radial nerve and musculocutaneous nerve are identified and labeled via respective fully colored segmentation masks and the location of which are tracked (spatially and temporally) over different frames (i.e., FIG. 27A to FIG. 27B to FIG. 27C).

Automated Labeling

The nature of the intervention (i.e. nerve blocking) requires one to identify and segment the different classes of nerve in real time. The systems and methods of the present invention make use of a machine learning pipeline (pre-processing module, machine learning algorithm and post-processing) fully integrated and optimized for real time segmentation.

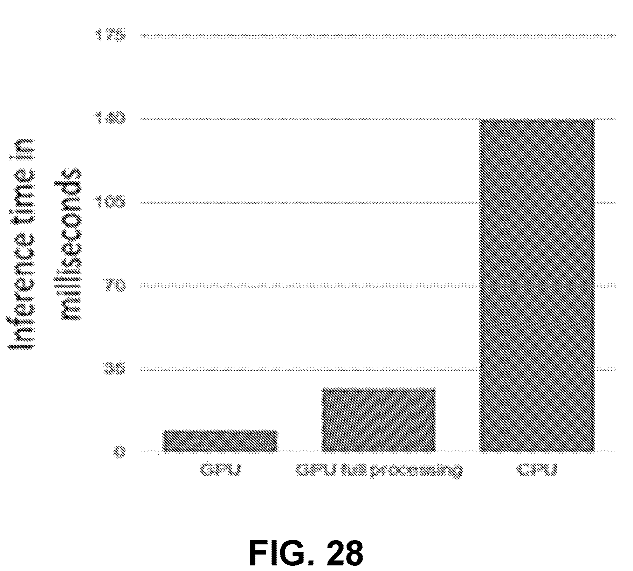

For example, as illustrated in FIG. 28, the inference time for a single frame may be 0.14 seconds average on an Intel Cascade Lake CPU and 0.009 seconds average on NVIDIA T4 GPU, whereas the total preprocessing, inference and post processing time for a single frame is 0.027 sec on the same GPU, thus making us able to entirely process frames with 30 frames per second (fps). Accordingly, the model inference time on GPU is small enough to consider processing each frame of the US film within the frame refreshment time.

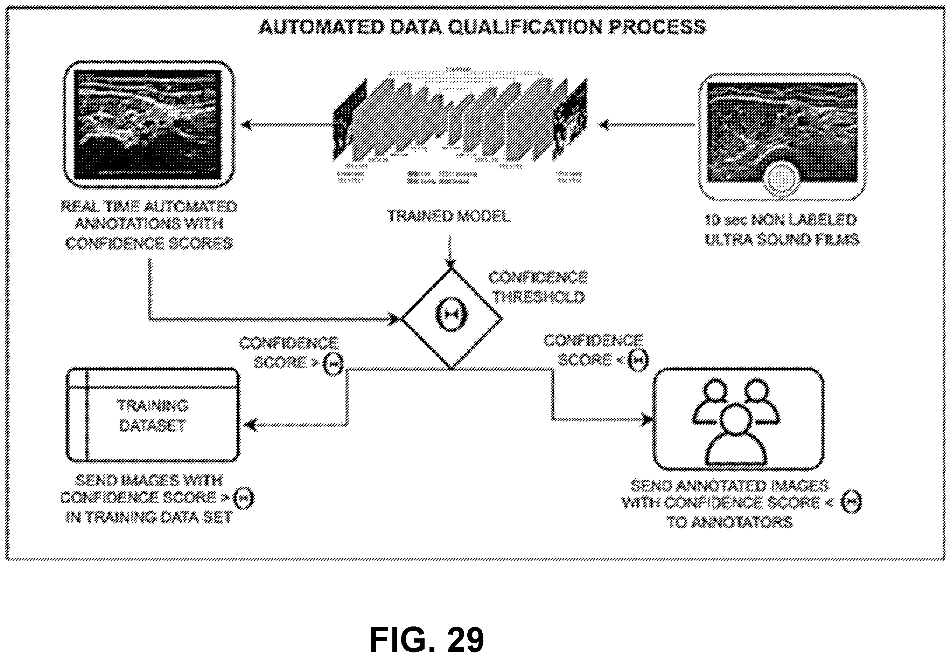

The process of manual annotation can be laborious and expensive for professionals (the anesthetists). As such, in order to improve the annotation process and reduce the effort and time of the professional annotators, the present invention further includes a means for automating the annotation process by selecting a sufficient number of manually annotated data to train a model which can automatically detect and segment the different nerves + artery in real time. Once the model achieves a reasonable level of performance, a confidence score threshold can be defined during the evaluation phase, wherein above the threshold the nerve segmentation can be considered acceptable and below the threshold the annotations can be rejected. The trained model is then used to automate the annotations of the image frames of the US films, which will come with a confidence score. The image confidence score will be compared to the confidence score threshold and the image will be directed either to the training dataset (if the confidence score > confidence score threshold) or to the annotators to be reviewed and corrected (if the confidence score < confidence score threshold). The process of the automated labeling framework is presented in FIG. 29.

Accordingly, the tissue detection and tracking model provided by systems and methods of the present invention provides numerous advantages. In particular, the model is trained to automatically describe tissue and organs encountered during an ultrasound examination, thereby allowing for the detection and tracking tissue characteristics for the specific use related to anatomical topography via a semantic segmentation, specifically the automatic detection of axillary nerves for upper limb operations without general anesthesia, referred to herein as "nerve cartography". The model is able to address at least two problems, including the data acquisition and qualification process, as well as the exploitation of the semantic segmentation model to produce and track, via crosses, the necessary information to super-impose onto the successive images making an US film.

As used in any embodiment herein, the term "module" may refer to software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices. "Circuitry", as used in any embodiment herein, may comprise, for example, singly or in any combination, hardwired circuitry, programmable circuitry such as computer processors comprising one or more individual instruction processing cores, state machine circuitry, and/or firmware that stores instructions executed by programmable circuitry. The modules may, collectively or individually, be embodied as circuitry that forms part of a larger system, for example, an integrated circuit (IC), system on-chip (SoC), desktop computers, laptop computers, tablet computers, servers, smartphones, etc.

Any of the operations described herein may be implemented in a system that includes one or more storage mediums having stored thereon, individually or in combination, instructions that when executed by one or more processors perform the methods. Here, the processor may include, for example, a server CPU, a mobile device CPU, and/or other programmable circuitry.

Also, it is intended that operations described herein may be distributed across a plurality of physical devices, such as processing structures at more than one different physical location. The storage medium may include any type of tangible medium, for example, any type of disk including hard disks, floppy disks, optical disks, compact disk read-only memories (CD-ROMs), compact disk rewritables (CD-RWs), and magneto-optical disks, semiconductor devices such as read-only memories (ROMs), random access memories (RAMs) such as dynamic and static RAMs, erasable programmable read-only memories (EPROMs), electrically erasable programmable read-only memories (EEPROMs), flash memories, Solid State Disks (SSDs), magnetic or optical cards, or any type of media suitable for storing electronic instructions. Other embodiments may be implemented as software modules executed by a programmable control device. The storage medium may be non-transitory.

As described herein, various embodiments may be implemented using hardware elements, software elements, or any combination thereof. Examples of hardware elements may include processors, microprocessors, circuits, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth.

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The invention is defined by the appended claims.

**Claims**

1. A system for providing automated medical imaging analysis, the system comprising:
a computing system comprising a hardware processor coupled to non-transitory, computer-readable memory containing instructions executable by the processor to cause the computing system to:

run a neural network, wherein the neural network has been trained using a plurality of training data sets, each training data set comprises reference ultrasound image data associated with known tissue and/or anatomical structures and known condition data associated with the known tissue and/or anatomical structures;
receive and analyze a sample ultrasound image of a target site of a patient by using the neural network and based on an association of the condition data with the reference ultrasound image data;
identify, based on the analysis, one or more types of tissue and/or anatomical structures and an associated condition of the one or more types of tissue and/or anatomical structures in the sample ultrasound image; and
output, via a display, an augmented ultrasound image comprising a visual representation of the one or more identified types of tissue and/or anatomical structures at the target site of the patient and an associated abnormal condition of the one or more identified types of tissue and/or anatomical structures, if present,
wherein the visual representation comprises at least a first layer of content overlaid upon the sample ultrasound image and a second layer of content overlaid upon the sample ultrasound image,

wherein the first layer of content comprises semantic segmentation of different types of tissue within the sample ultrasound image and/or different anatomical structures within the sample ultrasound image,
wherein the second layer of content comprises a visual indication of an abnormal condition associated with the one or more identified types of tissue and/or identified anatomical structures, and
wherein the first layer of content provides semantic segmentation of the various organs, including segmentation of the liver, gallbladder and digestive structure, and the second layer of content provides an indication of the presence of gallstones in the gallbladder.

2. The system of claim 1, wherein the first layer of content comprises one or more shaded zones overlaid upon one or more respective portions of the sample ultrasound image.

3. The system of claim 2, wherein each of the one or more shaded zones corresponds to a respective one of the identified types of tissue and/or identified anatomical structures.

4. The system of claim 3, wherein each of the identified types of tissue and/or identified anatomical structures comprises a shaded zone with a distinct color and/or pattern to distinguish from one another.

5. The system of claim 3, wherein the first layer of content further comprises text associated with each of the one or more shaded zones, wherein the text identifies the respective one of the identified types of tissue and/or identified anatomical structures.

6. The system of claim 1, wherein the visual indication comprises text identifying the specific abnormal condition.

7. The system of claim 6, wherein the visual indication comprises a marking and/or shaded zone corresponding to at least a portion of one of the identified types of tissue and/or identified anatomical structures to which the abnormal condition is associated.

8. The system of claim 1, wherein the abnormal condition comprises a structural abnormality or a disease.

9. The system of claim 1, wherein the analysis of the sample ultrasound image comprises correlating sample image data with the known tissue and/or anatomical structures of the reference ultrasound image data and known condition data associated therewith.

10. The system of claim 1, wherein the computing system comprises a machine learning system selected from the group consisting of a neural network, a random forest, a support vector machine, a Bayesian classifier, a Hidden Markov model, an independent component analysis method, and a clustering method.

11. The system of claim 1, wherein the computing system comprises an autonomous machine learning system that associates the condition data with the reference ultrasound image data.

12. The system of claim 11, wherein the machine learning system comprises a deep learning neural network that includes an input layer, a plurality of hidden layers, and an output layer.

13. The system of claim 12, wherein the autonomous machine learning system represents the training data set using a plurality of features, wherein each feature comprises a feature vector.

14. The system of claim 12, wherein the autonomous machine learning system comprises a convolutional neural network (CNN).

15. The system of claim 1, wherein the reference ultrasound image data and condition data are obtained from one or more third party sources, wherein the third party sources comprises publicly available or subscription-based data sources.

**Patentansprüche**

1. System zum Bereitstellen einer automatisierten medizinischen Bildgebungsanalyse, wobei das System Folgendes umfasst:

ein Rechensystem, das einen Hardwareprozessor umfasst, der an einen nichtflüchtigen, computerlesbaren Speicher gekoppelt ist, der Anweisungen enthält, die durch den Prozessor ausführbar sind, um das Rechensystem zu Folgendem zu veranlassen:

Betreiben eines neuronalen Netzes, wobei das neuronale Netz unter Verwendung einer Vielzahl von Trainingsdatensätzen trainiert wurde, wobei jeder Trainingsdatensatz Referenzultraschallbilddaten, die bekanntem Gewebe und/oder bekannten anatomischen Strukturen zugeordnet sind, und bekannte Zustandsdaten, die dem bekannten Gewebe und/oder den bekannten anatomischen Strukturen zugeordnet sind, umfasst;
Empfangen und Analysieren eines Probenultraschallbilds eines Zielorts eines Patienten unter Verwendung des neuronalen Netzes und basierend auf einer Zuordnung der Zustandsdaten zu den Referenzultraschallbilddaten;
Identifizieren, basierend auf der Analyse, einer oder mehrerer Arten von Gewebe und/oder anatomischen Strukturen und eines zugeordneten Zustands der einen oder mehreren Arten von Gewebe und/oder anatomischen Strukturen in dem Probenultraschallbild; und
Ausgeben über eine Anzeige eines erweiterten Ultraschallbildes, das eine visuelle Darstellung des einen oder der mehreren identifizierten Arten von Gewebe und/oder anatomischen Strukturen an dem Zielort des Patienten und einen zugeordneten abnormalen Zustand des einen oder der mehreren identifizierten Arten von Gewebe und/oder anatomischen Strukturen, falls vorhanden, umfasst,
wobei die visuelle Darstellung mindestens eine erste Inhaltsschicht, die dem Probenultraschallbild überlagert ist, und eine zweite Inhaltsschicht, die dem Probenultraschallbild überlagert ist, umfasst,
wobei die erste Inhaltsschicht eine semantische Segmentierung verschiedener Arten von Gewebe innerhalb des Probenultraschallbilds und/oder verschiedener anatomischer Strukturen innerhalb des Probenultraschallbilds umfasst,
wobei die zweite Inhaltsschicht eine visuelle Angabe eines abnormalen Zustands umfasst, der dem einen oder den mehreren identifizierten Arten von Gewebe und/oder identifizierten anatomischen Strukturen zugeordnet ist, und
wobei die erste Inhaltsschicht eine semantische Segmentierung der verschiedenen Organe bereitstellt, einschließlich der Segmentierung der Leber-, Gallenblasen- und Verdauungsstruktur, und die zweite Inhaltsschicht eine Angabe des Vorhandenseins von Gallensteinen in der Gallenblase bereitstellt.

2. System nach Anspruch 1, wobei die erste Inhaltsschicht eine oder mehrere schraffierte Zonen umfasst, die einem oder mehreren jeweiligen Abschnitten des Probenultraschallbilds überlagert sind.

3. System nach Anspruch 2, wobei jede von der einen oder den mehreren schraffierten Zonen einem jeweiligen der identifizierten Arten von Gewebe und/oder identifizierten anatomischen Strukturen entspricht.

4.  System nach Anspruch 3, wobei jede der identifizierten Arten von Gewebe und/oder identifizierten anatomischen Strukturen eine schraffierte Zone mit einer eigenen Farbe und/oder einem eigenen Muster umfasst, um sie voneinander zu unterscheiden.

5.  System nach Anspruch 3, wobei die erste Inhaltsschicht ferner Text umfasst, der jeder von der einen oder den mehreren schraffierten Zonen zugeordnet ist, wobei der Text die jeweilige der identifizierten Arten von Gewebe und/oder identifizierten anatomischen Strukturen identifiziert.

6.  System nach Anspruch 1, wobei die visuelle Angabe Text umfasst, der den spezifischen abnormalen Zustand identifiziert.

7.  System nach Anspruch 6, wobei die visuelle Angabe eine Markierung und/oder schraffierte Zone umfasst, die mindestens einem Abschnitt einer der identifizierten Arten von Gewebe und/oder identifizierten anatomischen Strukturen entspricht, denen der abnormale Zustand zugeordnet ist.

8.  System nach Anspruch 1, wobei der abnormale Zustand eine strukturelle Abnormalität oder eine Erkrankung umfasst.

9.  System nach Anspruch 1, wobei die Analyse des Probenultraschallbilds Korrelieren von Probenbilddaten mit dem bekannten Gewebe und/oder den bekannten anatomischen Strukturen der Referenzultraschallbilddaten und den bekannten Zustandsdaten, die diesen zugeordnet sind, umfasst.

10. System nach Anspruch 1, wobei das Rechensystem ein maschinelles Lernsystem umfasst, das aus der Gruppe ausgewählt ist, die aus einem neuronalen Netz, einem Random Forest, einer Support Vector Machine, einem Bayes-Klassifikator, einem Hidden Markov Model, einem Verfahren der Unabhängigkeitsanalyse und einem Clustering-Verfahren besteht.

11. System nach Anspruch 1, wobei das Rechensystem ein autonomes maschinelles Lernsystem umfasst, das die Zustandsdaten den Referenzultraschallbilddaten zuordnet.

12. System nach Anspruch 11, wobei das maschinelle Lernsystem ein neuronales Deep-Learning-Netz umfasst, das eine Eingabeschicht, eine Vielzahl von verborgenen Schichten und eine Ausgabeschicht enthält.

13. System nach Anspruch 12, wobei das autonome maschinelle Lernsystem den Trainingsdatensatz unter Verwendung einer Vielzahl von Merkmalen darstellt, wobei jedes Merkmal einen Merkmalsvektor umfasst.

14. System nach Anspruch 12, wobei das autonome maschinelle Lernsystem ein Convolutional Neural Network (CNN) umfasst.

15. System nach Anspruch 1, wobei die Referenzultraschallbilddaten und Zustandsdaten von einer oder mehreren Drittquellen erhalten werden, wobei die Drittquellen öffentlich verfügbare oder abonnementbasierte Datenquellen umfassen.

**Revendications**

1.  Système permettant de fournir une analyse d'imagerie médicale automatisée, le système comprenant :
    un système informatique comprenant un processeur matériel couplé à une mémoire non transitoire lisible par ordinateur contenant des instructions exécutables par le processeur pour amener le système informatique à :

    exécuter un réseau neuronal, ledit réseau neuronal ayant été entraîné à l'aide d'une pluralité d'ensembles de données d'entraînement, chaque ensemble de données d'entraînement comprenant des données d'image ultrasonore de référence associées à un tissu et/ou des structures anatomiques connus et des données d'état connues associées au tissu et/ou aux structures anatomiques connus ;
    recevoir et analyser une image ultrasonore d'échantillon d'un site cible d'un patient à l'aide du réseau neuronal et sur la base d'une association des données d'état avec les données d'image ultrasonore de référence ;
    identifier, sur la base de l'analyse, un ou plusieurs types de tissu et/ou structures anatomiques et un état associé desdits un ou plusieurs types de tissu et/ou structures anatomiques dans l'image ultrasonore d'échantillon ; et

sortir, via un écran, une image ultrasonore augmentée comprenant une représentation visuelle desdits un ou plusieurs types identifiés de tissu et/ou structures anatomiques au niveau du site cible du patient et d'un état anormal associé desdits un ou plusieurs types de tissu identifiés et/ou de structures anatomiques, le cas échéant, ladite représentation visuelle comprenant au moins une première couche de contenu superposée sur l'image ultrasonore d'échantillon et une seconde couche de contenu superposée sur l'image ultrasonore d'échantillon,

ladite première couche de contenu comprenant une segmentation sémantique de différents types de tissu au sein de l'image ultrasonore d'échantillon et/ou de différentes structures anatomiques au sein de l'image ultrasonore d'échantillon,

ladite seconde couche de contenu comprenant une indication visuelle d'un état anormal associé auxdits un ou plusieurs types de tissu identifiés et/ou structures anatomiques identifiées, et

ladite première couche de contenu fournissant une segmentation sémantique des différents organes, y compris la segmentation du foie, de la vésicule biliaire et de la structure digestive, et ladite seconde couche de contenu fournissant une indication de la présence de calculs biliaires dans la vésicule biliaire.

2. Système selon la revendication 1, ladite première couche de contenu comprenant une ou plusieurs zones ombrées superposées sur une ou plusieurs parties respectives de l'image ultrasonore d'échantillon.

3. Système selon la revendication 2, chacune desdites une ou plusieurs zones ombrées correspondant à un type respectif des types de tissu identifiés et/ou structures anatomiques identifiées.

4. Système selon la revendication 3, chacun des types de tissu identifiés et/ou structures anatomiques identifiées comprenant une zone ombrée avec une couleur et/ou un motif distinct permettant de les distinguer les uns des autres.

5. Système selon la revendication 3, ladite première couche de contenu comprenant en outre un texte associé à chacune desdites une ou plusieurs zones ombrées, ledit texte identifiant le type respectif des types de tissu identifiés et/ou des structures anatomiques identifiées.

6. Système selon la revendication 1, ladite indication visuelle comprenant un texte identifiant l'état anormal spécifique.

7. Système selon la revendication 6, ladite indication visuelle comprenant un marquage et/ou une zone ombrée correspondant à au moins une partie de l'un des types de tissu identifiés et/ou des structures anatomiques identifiées auxquels l'état anormal est associé.

8. Système selon la revendication 1, ladite condition anormale comprenant une anomalie structurelle ou une maladie.

9. Système selon la revendication 1, ladite analyse de l'image ultrasonore d'échantillon comprenant la corrélation des données d'image d'échantillon avec le tissu et/ou les structures anatomiques connus des données d'image ultrasonore de référence et des données d'état connues associées à celles-ci.

10. Système selon la revendication 1, ledit système informatique comprenant un système d'apprentissage automatique choisi dans le groupe constitué par un réseau neuronal, une forêt aléatoire, une machine à vecteurs de support, un classifieur bayésien, un modèle de Markov caché, un méthode d'analyse de constituants indépendants et une méthode de regroupement.

11. Système selon la revendication 1, ledit système informatique comprenant un système d'apprentissage automatique autonome qui associe les données d'état aux données d'image ultrasonore de référence.

12. Système selon la revendication 11, ledit système d'apprentissage automatique comprenant un réseau neuronal d'apprentissage profond qui comprend une couche d'entrée, une pluralité de couches cachées et une couche de sortie.

13. Système selon la revendication 12, ledit système d'apprentissage automatique autonome représentant l'ensemble de données d'apprentissage en utilisant une pluralité de caractéristiques, chaque caractéristique comprenant un vecteur de caractéristiques.

14. Système selon la revendication 12, ledit système d'apprentissage automatique autonome comprenant un réseau neuronal convolutionnel (CNN).

15. Système selon la revendication 1, lesdites données d'image ultrasonore de référence et lesdites données d'état étant obtenues à partir d'une ou plusieurs sources tierces, lesdites sources tierces comprenant des sources de données accessibles au public ou basées sur un abonnement.

## Ultrasound Imaging Machine
### 10

> ### Ultrasound Device
> #### 12

> ### Ultrasound Console
> #### 14

> ### Ultrasound Display
> #### 16

## Automated Ultrasound Imaging Analysis System
### 100

# FIG. 1A

12

Patient

# FIG. 1B

Cloud-based Service
102

Automated Ultrasound Imaging
Analysis System
100

Network
104

Ultrasound
Imaging Machine
10

Computing
Device
11

User

# FIG. 2

Automated Ultrasound Imaging Analysis System
100

Reference
Database
112

Sample
Database
114

Machine Learning
System
108

Image Analysis and
Content Creation
Module
110

Interface
106

Network
104

# FIG. 3

116

Training data set
116

Known tissue and/
or anatomical
structure

US Image
data

Condition
Data

Machine Learning System
108

Reference
Database
112

# FIG. 4

**FIG. 5**

FIG. 6

FIG. 7

FIG. 8

EP 4 453 864 B1

100

Reference
Database
210

Sample
Database
212

Machine Learning System
108

Image Analysis and
Content Creation Module
110

Sample US
image(s)

US image
overlay content

# FIG. 9

**FIG. 10A**

**FIG. 10B**

FIG. 11

EP 4 453 864 B1

**FIG. 12**

FIG. 13

MODEL DEVELOPMENT

MODEL IN PRODUCTION

CLINICAL DATA CREATION FOR MODEL TRAINING

Data Labelling

Data Anonymisation

Data Extraction from Ultrasound Machine

MODEL TRAINING on HDH CERTIFIED ENVIRONMENT

Data Storage and Encryption

Data Treatment and Preparation for ML

Dataset Creation

ML Model Training

Performance metrics checking

INFERENCE EMBEDDED IN THE US MACHINE

US Machine

Data Anonymisation

Prediction

MODEL IMPROVEMENT

Verification

········▷ Data process flow during clinical validation

FIG. 14

EP 4 453 864 B1

FIG. 15

```
import shutil
import os
import sklearn.model_selection
from sklearn.model_selection import train_test_split

# Fonction de creation des dataset d'apprentissage et de test
def split_dataset (directory) :
  Data = []

# Split des fichiers en train_set, val_set et test_set
  pathlist = directory.glob("*_Gradient")
  for path in pathlist :
    Data.append(str(path))

  train_data, test_data = train_test_split(Data, test_size=0.1, random_state=1)
  train_data, val_data = train_test_split(train_data, test_size=0.2, random_state=1)

  for file in train_data:
    shutil.move(file, directory.parent.parent/'TRAIN_SET')
  for file in test_data:
    shutil.move(file, directory.parent.parent/'TEST_SET')
  for file in val_data:
    shutil.move(file, directory.parent.parent/'VAL_SET')

Y = split_dataset(Path("SOURCE/Malin"))
Y = split_dataset(Path("SOURCE/BeninN"))
Y = split_dataset(Path("SOURCE/BeninK"))
Y = split_dataset(Path("SOURCE/Normal"))
Y = split_dataset(Path("SOURCE/Ganglion"))
Y = split_dataset(Path("SOURCE/Adenopathie"))
```

# FIG. 16

```
# Fonction de répartition des fichiers pour L4V (anomaly/normal)
def distri_s3 (directory) :
  Adeno = []
  Benin = []
  Malin = []
  Normal = []
  Ganglion = []

  pathlist = directory.glob("ADENO_*")
  for path in pathlist :
    Adeno.append(str(path))
  for file in Adeno:
    shutil.move(file, directory/'normal')

  pathlist = directory.glob("BENIN_*")
  for path in pathlist :
    Benin.append(str(path))
  for file in Benin:
    shutil.move(file, directory/'normal')

  pathlist = directory.glob("GANGL_*")
  for path in pathlist :
    Ganglion.append(str(path))
  for file in Ganglion:
    shutil.move(file, directory/'normal')

  pathlist = directory.glob("NORMA_*")
  for path in pathlist :
    Normal.append(str(path))
  for file in Normal:
    shutil.move(file, directory/'normal')

  pathlist = directory.glob("MALIN_*")
  for path in pathlist :
    Malin.append(str(path))
  for file in Malin:
    shutil.move(file, directory/'anomaly')

Z = distri_s3(Path("TRAIN_SET"))
Z = distri_s3(Path("VAL_SET"))
Z = distri_s3(Path("TEST_SET"))
```

# FIG. 17

```
import matplotlib.pyplot as plt
from PIL import Image
from pathlib import Path

# Fonction de lecture et travail sur les images pour constitution dataset
def get_corpus_video(directory) :
    Dataset=[]

# Lecture des fichiers dans les répertoires
    pathlist = directory.glob("*/*/*.jpeg")
    lst = list(pathlist)
    for path in lst [ : :2] :
        print(path)
        category = path.parent.name[0:11]
        count = path.name[6:10]
        print(category)
        file_final = f"{str(category)}_GradVid-{str(count)}.jpg"

# Travail de standardisation des images
        with Image.open(path) as im:
            plt.imshow(im)
            plt.show()
            width, height = im.size
            print(width, height)
            left = 170
            top = 100
            right = 650
            bottom = 540
            im1 = im.crop((left, top, right, bottom))
            plt.imshow(im1)
            plt.show()
            width, height = im1.size
            print(width, height)
            im1.save(directory/file_final)

X = get_corpus_video(Path("TRAIN_SET"))
X = get_corpus_video(Path("VAL_SET"))
X = get_corpus_video(Path("TEST_SET"))
```

# FIG. 18

Prediction - Anomaly class _ 77 images analyzed

$Prediction_{median} = 0,62$
$Prediction_{average} = 0,57$
$Prediction_{uncertainty} = 0,22$

FIG. 19A

FIG. 19B

EP 4 453 864 B1

Input ultrasound film (20 secs)

Input Phase

N frames extraction and format standardization

Temporary Frames Storage

Data preparation before inferring the model defined during the training phase

Model inference for image $i \in [1,N]$

Model output

The individual image or frame is analyzed by the model

Activation function

Individual Prediction score ($Pred_{i\_k} \in [0, 1]$) for $i \in [1,N]$ and for each k class (anomaly and normal)

Have all the N frames been analyzed?

NO

YES

Compute prediction score for each individual image

Activation function – Sigmoid type

FIG. 20

EP 4 453 864 B1

Have all the N frames been analyzed?

↓ YES

Average and
Uncertainty Function

Average Prediction score ($\mu_{pred\_k} \in [0, 1]$) over the N frames prediction and for each k class with k = 2

Uncertainty score ($\sigma_{pred\_k}$) over the N frames prediction and for each k class with k = 2

Compute average prediction score and uncertainty at patient level

ArgMax ($\mu_{pred\_k}$) for k = 2

ArgMax function returns the class having the highest value

Prediction

Or

k = 1          k = 2

Anomaly        Normal

Degree of Belief $\sigma_{pred\_k}$ with k = ArgMax ($\mu_{pred\_k}$)

Final outcome at patient level: prediction and degree of belief

$\sigma_{pred} < \sigma_{thre}$? → NO → Prediction Rejected

↓ YES

Prediction Acceptable

Acceptability of the prediction

FIG. 20 (cont.)

EP 4 453 864 B1

**FIG. 21A**

FIG. 21B

FIG. 22

FIG. 23

Concatenate

256 x 256

512 x 512

128 x 128

64 x 64

32 x 32

64 x 64

256 x 256

512 x 512

N-band input
512 x 512

Conv
Pooling

UpSampling
Dropout

UNet output
512 x 512

## FIG. 24

EP 4 453 864 B1

FIG. 25A    FIG. 25B    FIG. 25C

**FIG. 26**

# FIG. 27A
# FIG. 27B
# FIG. 27C

-+- Artery    -+- Median    -+- Ulnar    -+- Radial    -+- Musculocutaneous

EP 4 453 864 B1

**FIG. 28**

AUTOMATED DATA QUALIFICATION PROCESS

10 sec NON LABELED ULTRA SOUND FILMS

REAL TIME AUTOMATED ANNOTATIONS WITH CONFIDENCE SCORES

TRAINED MODEL

CONFIDENCE THRESHOLD

CONFIDENCE SCORE >

CONFIDENCE SCORE <

SEND IMAGES WITH CONFIDENCE SCORE > IN TRAINING DATA SET

TRAINING DATASET

SEND ANNOTATED IMAGES WITH CONFIDENCE SCORE < TO ANNOTATORS

**FIG. 29**

**REFERENCES CITED IN THE DESCRIPTION**

**Patent documents cited in the description**

- US 63292764
- US 63310761
- CN 110599476
- CN 111754530